# EUROPEAN PATENT APPLICATION

(11) **EP 3 957 364 A1**
(43) Date of publication of application: **23.02.2022**
(21) Application number: 20191900.8
(22) Date of filing: 20.08.2020
(51) Int. Cl.: A61P 11/00, A61K 39/395

(54) **A BINDING MOLECULE SPECIFICALLY BINDING TO HUMAN CD4 FOR USE IN THE TREATMENT OF PULMONARY DISEASES**

(71) Applicant: Fraunhofer-Gesellschaft zur Förderung der angewandten Forschung e.V., 80686 München (DE)
(72) Inventor: FRICKE, Stephan., 04103 Leipzig (DE); BRAUN, Armin., 30625 Hannover (DE); BLAUDSZUN, André-René., 04103 Leipzig (DE); SACK, Ulrich., 04275 Leipzig (DE)
(74) Representative: Pintsch, Tanja

(57) **Abstract**

The present invention relates to a binding molecule specifically binding to human CD4 for use in the prevention, amelioration and/or treatment of at least symptom of a disease associated with inflammation of the respiratory tract and/or immunologically induced injury of the respiratory tract in a human subject, wherein the binding molecule is administered intranasally and/or via inhalation and/or systemically, as well as to pharmaceutical compositions, pulmonary drug delivery kits and pharmaceutical packages related thereto.

SEQUENCE LISTING

<110>  Fraunhofer-Gesellschaft zur Förderung der angewandten
       Forschung e.V.

<120>  A binding molecule specifically binding to human CD4 for use in the
       treatment of pulmonary diseases

<130>  F73083EP

<160>  21

<170>  PatentIn version 3.5

<210>  1
<211>  360
<212>  DNA
<213>  Artificial Sequence

<220>
<223>  Mouse Anti-CD4 Heavy Chain

<220>
<221>  CDS
<222>  (1)..(360)

<400>  1
gag gtt cag ctc cag cag tct ggg act gtg ctg gca agg cct ggg gct        48
Glu Val Gln Leu Gln Gln Ser Gly Thr Val Leu Ala Arg Pro Gly Ala
1               5                   10                  15

tcc gtg cag atg tcc tgc aag gct tct ggc tac agc ttt gcc aac tac        96
Ser Val Gln Met Ser Cys Lys Ala Ser Gly Tyr Ser Phe Ala Asn Tyr
            20                  25                  30

tgg atg cac tgg gta aaa cag agg cct gga cag ggt cta caa tgg att       144
Trp Met His Trp Val Lys Gln Arg Pro Gly Gln Gly Leu Gln Trp Ile
        35                  40                  45

ggt gct ctt tat cct gga aat gtt gat act acc tac aac cag aag ttc       192
Gly Ala Leu Tyr Pro Gly Asn Val Asp Thr Thr Tyr Asn Gln Lys Phe
    50                  55                  60

aag gac aag gcc aaa ctg act gca gtc aca tcc gcc agc act gcc tac       240
Lys Asp Lys Ala Lys Leu Thr Ala Val Thr Ser Ala Ser Thr Ala Tyr
65                  70                  75                  80

atg gag ctc agc agc ctg aca aat gag gac tct gcg gtc tat tac tgt       288
Met Glu Leu Ser Ser Leu Thr Asn Glu Asp Ser Ala Val Tyr Tyr Cys
                85                  90                  95

aca aga atg ggt act act tta gaa gcc ccc ctt gac tat tgg ggc caa       336
Thr Arg Met Gly Thr Thr Leu Glu Ala Pro Leu Asp Tyr Trp Gly Gln
            100                 105                 110

ggc acc act ctc aca gtc tcc tca                                       360
Gly Thr Thr Leu Thr Val Ser Ser
        115                 120

<210>  2
<211>  120

<212>  PRT
<213>  Artificial Sequence

<220>
<223>  Synthetic Construct

<400>  2

Glu Val Gln Leu Gln Gln Ser Gly Thr Val Leu Ala Arg Pro Gly Ala
1               5                   10                  15

Ser Val Gln Met Ser Cys Lys Ala Ser Gly Tyr Ser Phe Ala Asn Tyr
            20                  25                  30

Trp Met His Trp Val Lys Gln Arg Pro Gly Gln Gly Leu Gln Trp Ile
        35                  40                  45

Gly Ala Leu Tyr Pro Gly Asn Val Asp Thr Thr Tyr Asn Gln Lys Phe
    50                  55                  60

Lys Asp Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Met Gly Thr Thr Leu Glu Ala Pro Leu Asp Tyr Trp Gly Gln
            100                 105                 110

Gly Thr Thr Leu Thr Val Ser Ser
        115                 120


<210>  3
<211>  360
<212>  DNA
<213>  Artificial Sequence

<220>
<223>  Heavy chain VH4


<220>
<221>  CDS
<222>  (1)..(360)

<400>  3
gag gtt cag ctc cag cag tct ggg act gag ctg aaa agg cct ggg gct       48
Glu Val Gln Leu Gln Gln Ser Gly Thr Glu Leu Lys Arg Pro Gly Ala
1               5                   10                  15

tcc gtg aag atg tcc tgc aag gct tct ggc tac acc ttt gcc aac tac       96
Ser Val Lys Met Ser Cys Lys Ala Ser Gly Tyr Thr Phe Ala Asn Tyr
            20                  25                  30

tgg atg cac tgg gta aaa cag gcc cct gga cag ggt cta caa tgg att      144

Trp Met His Trp Val Lys Gln Ala Pro Gly Gln Gly Leu Gln Trp Ile
        35                  40                  45

ggt gct ctt tat cct gga aat gtt gat act acc tac aac cag aag ttc        192
Gly Ala Leu Tyr Pro Gly Asn Val Asp Thr Thr Tyr Asn Gln Lys Phe
    50                  55                  60

aag gac aag gcc aaa ctg act gca gtc aca tcc gcc agc act gcc tac        240
Lys Asp Lys Ala Lys Leu Thr Ala Val Thr Ser Ala Ser Thr Ala Tyr
65                  70                  75                  80

atg gag ctc agc agc ctg aca aat gag gac tct gcg gtc tat tac tgt        288
Met Glu Leu Ser Ser Leu Thr Asn Glu Asp Ser Ala Val Tyr Tyr Cys
                85                  90                  95

aca aga atg ggt act act tta gaa gcc ccc ctt gac tat tgg ggc caa        336
Thr Arg Met Gly Thr Thr Leu Glu Ala Pro Leu Asp Tyr Trp Gly Gln
            100                 105                 110

ggc acc act gtc aca gtc tcc tca                                        360
Gly Thr Thr Val Thr Val Ser Ser
        115                 120

<210>  4
<211>  120
<212>  PRT
<213>  Artificial Sequence

<220>
<223>  Synthetic Construct

<400>  4

Glu Val Gln Leu Gln Gln Ser Gly Thr Glu Leu Lys Arg Pro Gly Ala
1               5                   10                  15

Ser Val Lys Met Ser Cys Lys Ala Ser Gly Tyr Thr Phe Ala Asn Tyr
            20                  25                  30

Trp Met His Trp Val Lys Gln Ala Pro Gly Gln Gly Leu Gln Trp Ile
        35                  40                  45

Gly Ala Leu Tyr Pro Gly Asn Val Asp Thr Thr Tyr Asn Gln Lys Phe
    50                  55                  60

Lys Asp Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Met Gly Thr Thr Leu Glu Ala Pro Leu Asp Tyr Trp Gly Gln
            100                 105                 110

Gly Thr Thr Val Thr Val Ser Ser

115                 120

<210>  5
<211>  360
<212>  DNA
<213>  Artificial Sequence

<220>
<223>  Heavy chain VH3

<220>
<221>  CDS
<222>  (1)..(360)

<400>  5
gag gtt cag ctc cag cag tct ggg tct gag ctg aaa agg cct ggg gct        48
Glu Val Gln Leu Gln Gln Ser Gly Ser Glu Leu Lys Arg Pro Gly Ala
1               5                   10                  15

tcc gtg aag atg tcc tgc aag gct tct ggc tac acc ttt gcc aac tac        96
Ser Val Lys Met Ser Cys Lys Ala Ser Gly Tyr Thr Phe Ala Asn Tyr
            20                  25                  30

tgg atg cac tgg gta aaa cag gcc cct gga cag ggt cta caa tgg att       144
Trp Met His Trp Val Lys Gln Ala Pro Gly Gln Gly Leu Gln Trp Ile
        35                  40                  45

ggt gct ctt tat cct gga aat gtt gat act acc tac aac cag aag ttc       192
Gly Ala Leu Tyr Pro Gly Asn Val Asp Thr Thr Tyr Asn Gln Lys Phe
    50                  55                  60

aag gac aag gcc aaa ctg act gca gtc aca tcc gcc agc act gcc tac       240
Lys Asp Lys Ala Lys Leu Thr Ala Val Thr Ser Ala Ser Thr Ala Tyr
65                  70                  75                  80

atg gag ctc agc agc ctg aca aat gag gac acc gcg gtc tat tac tgt       288
Met Glu Leu Ser Ser Leu Thr Asn Glu Asp Thr Ala Val Tyr Tyr Cys
                85                  90                  95

aca aga atg ggt act act tta gaa gcc ccc ctt gac tat tgg ggc caa       336
Thr Arg Met Gly Thr Thr Leu Glu Ala Pro Leu Asp Tyr Trp Gly Gln
            100                 105                 110

ggc acc ctt gtc aca gtc tcc tca                                       360
Gly Thr Leu Val Thr Val Ser Ser
        115                 120

<210>  6
<211>  120
<212>  PRT
<213>  Artificial Sequence

<220>
<223>  Synthetic Construct

<400>  6

Glu Val Gln Leu Gln Gln Ser Gly Ser Glu Leu Lys Arg Pro Gly Ala
1               5                   10                  15

Ser Val Lys Met Ser Cys Lys Ala Ser Gly Tyr Thr Phe Ala Asn Tyr
            20                  25                  30

Trp Met His Trp Val Lys Gln Ala Pro Gly Gln Gly Leu Gln Trp Ile
        35                  40                  45

Gly Ala Leu Tyr Pro Gly Asn Val Asp Thr Thr Tyr Asn Gln Lys Phe
    50                  55                  60

Lys Asp Lys Ala Lys Leu Thr Ala Val Thr Ser Ala Ser Thr Ala Tyr
65                  70                  75                  80

Met Glu Leu Ser Ser Leu Thr Asn Glu Asp Thr Ala Val Tyr Tyr Cys
                85                  90                  95

Thr Arg Met Gly Thr Thr Leu Glu Ala Pro Leu Asp Tyr Trp Gly Gln
            100                 105                 110

Gly Thr Leu Val Thr Val Ser Ser
        115                 120

<210> 7
<211> 360
<212> DNA
<213> Artificial Sequence

<220>
<223> Heavy chain VH2

<220>
<221> CDS
<222> (1)..(360)

<400> 7
gag gtt cag ctc cag cag tct ggg tct gag ctg aaa agg cct ggg gct 48
Glu Val Gln Leu Gln Gln Ser Gly Ser Glu Leu Lys Arg Pro Gly Ala
1               5                   10                  15

tcc gtg aag gtg tcc tgc aag gct tct ggc tac acc ttt gcc aac tac 96
Ser Val Lys Val Ser Cys Lys Ala Ser Gly Tyr Thr Phe Ala Asn Tyr
            20                  25                  30

tgg atg cac tgg gta aaa cag gcc cct gga cag ggt cta caa tgg att 144
Trp Met His Trp Val Lys Gln Ala Pro Gly Gln Gly Leu Gln Trp Ile
        35                  40                  45

ggt gct ctt tat cct gga aat gtt gat act acc tac aac cag aag ttc 192
Gly Ala Leu Tyr Pro Gly Asn Val Asp Thr Thr Tyr Asn Gln Lys Phe
    50                  55                  60

aag gac aag gcc aaa ctg act gca gac aca tcc gcc agc act gcc tac 240
Lys Asp Lys Ala Lys Leu Thr Ala Asp Thr Ser Ala Ser Thr Ala Tyr
65                  70                  75                  80

atg gag ctc agc agc ctg aca aat gag gac acc gcg gtc tat tac tgt     288
Met Glu Leu Ser Ser Leu Thr Asn Glu Asp Thr Ala Val Tyr Tyr Cys
                85                  90                  95

aca aga atg ggt act act tta gaa gcc ccc ctt gac tat tgg ggc caa     336
Thr Arg Met Gly Thr Thr Leu Glu Ala Pro Leu Asp Tyr Trp Gly Gln
            100                 105                 110

ggc acc ctt gtc aca gtc tcc tca                                     360
Gly Thr Leu Val Thr Val Ser Ser
        115                 120

<210>  8
<211>  120
<212>  PRT
<213>  Artificial Sequence

<220>
<223>  Synthetic Construct

<400>  8

Glu Val Gln Leu Gln Gln Ser Gly Ser Glu Leu Lys Arg Pro Gly Ala
1               5                   10                  15

Ser Val Lys Val Ser Cys Lys Ala Ser Gly Tyr Thr Phe Ala Asn Tyr
            20                  25                  30

Trp Met His Trp Val Lys Gln Ala Pro Gly Gln Gly Leu Gln Trp Ile
        35                  40                  45

Gly Ala Leu Tyr Pro Gly Asn Val Asp Thr Thr Tyr Asn Gln Lys Phe
    50                  55                  60

Lys Asp Lys Ala Lys Leu Thr Ala Asp Thr Ser Ala Ser Thr Ala Tyr
65                  70                  75                  80

Met Glu Leu Ser Ser Leu Thr Asn Glu Asp Thr Ala Val Tyr Tyr Cys
                85                  90                  95

Thr Arg Met Gly Thr Thr Leu Glu Ala Pro Leu Asp Tyr Trp Gly Gln
            100                 105                 110

Gly Thr Leu Val Thr Val Ser Ser
        115                 120

<210>  9
<211>  360
<212>  DNA
<213>  Artificial Sequence

<220>
<223>  Heavy chain VH1

<220>
<221>  CDS
<222>  (1)..(360)

<400>  9
gag gtt cag ctc cag cag tct ggg tct gag ctg aaa agg cct ggg gct      48
Glu Val Gln Leu Gln Gln Ser Gly Ser Glu Leu Lys Arg Pro Gly Ala
1               5                   10                  15

tcc gtg aag gtg tcc tgc aag gct tct ggc tac acc ttt gcc aac tac      96
Ser Val Lys Val Ser Cys Lys Ala Ser Gly Tyr Thr Phe Ala Asn Tyr
            20                  25                  30

tgg atg cac tgg gta aga cag gcc cct gga cag ggt cta caa tgg att      144
Trp Met His Trp Val Arg Gln Ala Pro Gly Gln Gly Leu Gln Trp Ile
        35                  40                  45

ggt gct ctt tat cct gga aat gtt gat act acc tac aac cag aag ttc      192
Gly Ala Leu Tyr Pro Gly Asn Val Asp Thr Thr Tyr Asn Gln Lys Phe
    50                  55                  60

aag gac aag gcc aaa atc act aga gac aca tcc gcc agc act gcc tac      240
Lys Asp Lys Ala Lys Ile Thr Arg Asp Thr Ser Ala Ser Thr Ala Tyr
65                  70                  75                  80

atg gag ctc agc agc ctg aca aat gag gac acc gcg gtc tat tac tgt      288
Met Glu Leu Ser Ser Leu Thr Asn Glu Asp Thr Ala Val Tyr Tyr Cys
                85                  90                  95

aca aga atg ggt act act tta gaa gcc ccc ctt gac tat tgg ggc caa      336
Thr Arg Met Gly Thr Thr Leu Glu Ala Pro Leu Asp Tyr Trp Gly Gln
            100                 105                 110

ggc acc ctt gtc aca gtc tcc tca                                      360
Gly Thr Leu Val Thr Val Ser Ser
        115                 120


<210>  10
<211>  120
<212>  PRT
<213>  Artificial Sequence

<220>
<223>  Synthetic Construct

<400>  10

Glu Val Gln Leu Gln Gln Ser Gly Ser Glu Leu Lys Arg Pro Gly Ala
1               5                   10                  15


Ser Val Lys Val Ser Cys Lys Ala Ser Gly Tyr Thr Phe Ala Asn Tyr
            20                  25                  30


Trp Met His Trp Val Arg Gln Ala Pro Gly Gln Gly Leu Gln Trp Ile
        35                  40                  45


Gly Ala Leu Tyr Pro Gly Asn Val Asp Thr Thr Tyr Asn Gln Lys Phe
    50                  55                  60

Lys Asp Lys Ala Lys Ile Thr Arg Asp Thr Ser Ala Ser Thr Ala Tyr
65                  70                  75                  80

Met Glu Leu Ser Ser Leu Thr Asn Glu Asp Thr Ala Val Tyr Tyr Cys
                85                  90                  95

Thr Arg Met Gly Thr Thr Leu Glu Ala Pro Leu Asp Tyr Trp Gly Gln
            100                 105                 110

Gly Thr Leu Val Thr Val Ser Ser
        115                 120

<210>  11
<211>  318
<212>  DNA
<213>  Artificial Sequence

<220>
<223>  Mouse Anti-CD4 Light Chain

<220>
<221>  CDS
<222>  (1)..(318)

<400>  11
caa att gtt ctc acc cag tct cca gca atc atg tct gca tct cca ggg      48
Gln Ile Val Leu Thr Gln Ser Pro Ala Ile Met Ser Ala Ser Pro Gly
1               5                   10                  15

gag aag gtc gcc atg acc tgc agt gcc agg tca agt gta agt tac ttg      96
Glu Lys Val Ala Met Thr Cys Ser Ala Arg Ser Ser Val Ser Tyr Leu
            20                  25                  30

tac tgg tac cag cag aag cca gga tcc tcc ccc aga ctc ctg att tat      144
Tyr Trp Tyr Gln Gln Lys Pro Gly Ser Ser Pro Arg Leu Leu Ile Tyr
        35                  40                  45

gac aca tcc aac ctg gct tct gga gtc cct gtt cgc ttc att ggc agt      192
Asp Thr Ser Asn Leu Ala Ser Gly Val Pro Val Arg Phe Ile Gly Ser
    50                  55                  60

ggg tct ggg acc tct tac tct ctc aca atc agc cga atg gag gct gaa      240
Gly Ser Gly Thr Ser Tyr Ser Leu Thr Ile Ser Arg Met Glu Ala Glu
65                  70                  75                  80

gat gct gcc act tat tac tgc cag cag tgg agt gat tac ccg ctc acg      288
Asp Ala Ala Thr Tyr Tyr Cys Gln Gln Trp Ser Asp Tyr Pro Leu Thr
                85                  90                  95

ttc ggt gct ggg acc aag ctg gag ctg aaa                              318
Phe Gly Ala Gly Thr Lys Leu Glu Leu Lys
            100                 105

<210>  12
<211>  106

<212>  PRT
<213>  Artificial Sequence

<220>
<223>  Synthetic Construct

<400>  12

Gln Ile Val Leu Thr Gln Ser Pro Ala Ile Met Ser Ala Ser Pro Gly
1               5                   10                  15

Glu Lys Val Ala Met Thr Cys Ser Ala Arg Ser Ser Val Ser Tyr Leu
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Val Arg Phe Ile Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Asp Tyr Pro Leu Thr
                85                  90                  95

Phe Gly Ala Gly Thr Lys Leu Glu Leu Lys
            100                 105


<210>  13
<211>  318
<212>  DNA
<213>  Artificial Sequence

<220>
<223>  Light Chain VK4


<220>
<221>  CDS
<222>  (1)..(318)

<400>  13
caa att gtt ctc acc cag tct cca gca atc ctg tct gca tct cca ggg      48
Gln Ile Val Leu Thr Gln Ser Pro Ala Ile Leu Ser Ala Ser Pro Gly
1               5                   10                  15

gag aag gcc gcc atg acc tgc agt gcc agg tca agt gta agt tac ttg      96
Glu Lys Ala Ala Met Thr Cys Ser Ala Arg Ser Ser Val Ser Tyr Leu
            20                  25                  30

tac tgg tac cag cag aag cca ggg tcc tcc ccc aga ctc ctg att tat      144
Tyr Trp Tyr Gln Gln Lys Pro Gly Ser Ser Pro Arg Leu Leu Ile Tyr
        35                  40                  45

gac aca tcc aac ctg gct tct gga gtc cct tct cgc ttc att ggc agt      192

Asp Thr Ser Asn Leu Ala Ser Gly Val Pro Ser Arg Phe Ile Gly Ser
    50                  55                  60

ggg tct ggg acc gat tac tct ctc aca atc agc agc atg gag gct gaa         240
Gly Ser Gly Thr Asp Tyr Ser Leu Thr Ile Ser Ser Met Glu Ala Glu
65                  70                  75                  80

gat gct gcc act tat tac tgc cag cag tgg agt gat tac ccg ctc acg         288
Asp Ala Ala Thr Tyr Tyr Cys Gln Gln Trp Ser Asp Tyr Pro Leu Thr
                85                  90                  95

ttc ggt gct ggg acc aag ctg gag atc aaa                                 318
Phe Gly Ala Gly Thr Lys Leu Glu Ile Lys
            100                 105

<210>  14
<211>  106
<212>  PRT
<213>  Artificial Sequence

<220>
<223>  Synthetic Construct

<400>  14

Gln Ile Val Leu Thr Gln Ser Pro Ala Ile Leu Ser Ala Ser Pro Gly
1               5                   10                  15

Glu Lys Ala Ala Met Thr Cys Ser Ala Arg Ser Ser Val Ser Tyr Leu
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Ser Arg Phe Ile Gly Ser
    50                  55                  60

Gly Ser Gly Thr Asp Tyr Ser Leu Thr Ile Ser Ser Met Glu Ala Glu
65                  70                  75                  80

Asp Ala Ala Thr Tyr Tyr Cys Gln Gln Trp Ser Asp Tyr Pro Leu Thr
                85                  90                  95

Phe Gly Ala Gly Thr Lys Leu Glu Ile Lys
            100                 105

<210>  15
<211>  318
<212>  DNA
<213>  Artificial Sequence

<220>
<223>  Light chain VK3

<220>
<221>  CDS
<222>  (1)..(318)

<400>  15
caa att gtt ctc acc cag tct cca gca acc ctg tct gca tct cca ggg      48
Gln Ile Val Leu Thr Gln Ser Pro Ala Thr Leu Ser Ala Ser Pro Gly
1               5                   10                  15

gag aag gcc gcc atg acc tgc agt gcc agg tca agt gta agt tac ttg      96
Glu Lys Ala Ala Met Thr Cys Ser Ala Arg Ser Ser Val Ser Tyr Leu
            20                  25                  30

tac tgg tac cag cag aag cca ggg tcc tcc ccc aga ctc ctg att tat      144
Tyr Trp Tyr Gln Gln Lys Pro Gly Ser Ser Pro Arg Leu Leu Ile Tyr
        35                  40                  45

gac aca tcc aac ctg gct tct gga gtc cct tct cgc ttc att ggc agt      192
Asp Thr Ser Asn Leu Ala Ser Gly Val Pro Ser Arg Phe Ile Gly Ser
    50                  55                  60

ggg tct ggg acc gat tac tct ctc aca atc agc agc atg gag gct gaa      240
Gly Ser Gly Thr Asp Tyr Ser Leu Thr Ile Ser Ser Met Glu Ala Glu
65                  70                  75                  80

gat gct gcc act tat tac tgc cag cag tgg agt gat tac ccg ctc acg      288
Asp Ala Ala Thr Tyr Tyr Cys Gln Gln Trp Ser Asp Tyr Pro Leu Thr
                85                  90                  95

ttc ggt gct ggg acc aag ctg gag atc aaa                              318
Phe Gly Ala Gly Thr Lys Leu Glu Ile Lys
            100                 105


<210>  16
<211>  106
<212>  PRT
<213>  Artificial Sequence

<220>
<223>  Synthetic Construct

<400>  16

Gln Ile Val Leu Thr Gln Ser Pro Ala Thr Leu Ser Ala Ser Pro Gly
1               5                   10                  15


Glu Lys Ala Ala Met Thr Cys Ser Ala Arg Ser Ser Val Ser Tyr Leu
            20                  25                  30


Tyr Trp Tyr Gln Gln Lys Pro Gly Ser Ser Pro Arg Leu Leu Ile Tyr
        35                  40                  45


Asp Thr Ser Asn Leu Ala Ser Gly Val Pro Ser Arg Phe Ile Gly Ser
    50                  55                  60


Gly Ser Gly Thr Asp Tyr Ser Leu Thr Ile Ser Ser Met Glu Ala Glu
65                  70                  75                  80

Asp Ala Ala Thr Tyr Tyr Cys Gln Gln Trp Ser Asp Tyr Pro Leu Thr
                85                  90                  95

Phe Gly Ala Gly Thr Lys Leu Glu Ile Lys
            100                 105

<210>  17
<211>  318
<212>  DNA
<213>  Artificial Sequence

<220>
<223>  Light chain VK2

<220>
<221>  CDS
<222>  (1)..(318)

<400>  17
caa att gtt ctc acc cag tct cca gca acc ctg tct gca tct cca ggg      48
Gln Ile Val Leu Thr Gln Ser Pro Ala Thr Leu Ser Ala Ser Pro Gly
1               5                   10                  15

gag aag gcc gcc atg acc tgc agt gcc agg tca agt gta agt tac ttg      96
Glu Lys Ala Ala Met Thr Cys Ser Ala Arg Ser Ser Val Ser Tyr Leu
            20                  25                  30

tac tgg tac cag cag aag cca ggg tcc tcc ccc aga ctc ctg att tat      144
Tyr Trp Tyr Gln Gln Lys Pro Gly Ser Ser Pro Arg Leu Leu Ile Tyr
        35                  40                  45

gac aca tcc aac ctg gct tct gga gtc cct tct cgc ttc agc ggc agt      192
Asp Thr Ser Asn Leu Ala Ser Gly Val Pro Ser Arg Phe Ser Gly Ser
    50                  55                  60

ggg tct ggg acc gat tac tct ctc aca atc agc agc atg gag gct gaa      240
Gly Ser Gly Thr Asp Tyr Ser Leu Thr Ile Ser Ser Met Glu Ala Glu
65                  70                  75                  80

gat gct gcc act tat tac tgc cag cag tgg agt gat tac ccg ctc acg      288
Asp Ala Ala Thr Tyr Tyr Cys Gln Gln Trp Ser Asp Tyr Pro Leu Thr
                85                  90                  95

ttc ggt gct ggg acc aag ctg gag atc aaa                              318
Phe Gly Ala Gly Thr Lys Leu Glu Ile Lys
            100                 105

<210>  18
<211>  106
<212>  PRT
<213>  Artificial Sequence

<220>
<223>  Synthetic Construct

<400>  18

Gln Ile Val Leu Thr Gln Ser Pro Ala Thr Leu Ser Ala Ser Pro Gly

1               5                   10                  15

Glu Lys Ala Ala Met Thr Cys Ser Ala Arg Ser Ser Val Ser Tyr Leu
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Ser Arg Phe Ser Gly Ser
    50                  55                  60

Gly Ser Gly Thr Asp Tyr Ser Leu Thr Ile Ser Ser Met Glu Ala Glu
65                  70                  75                  80

Asp Ala Ala Thr Tyr Tyr Cys Gln Gln Trp Ser Asp Tyr Pro Leu Thr
                85                  90                  95

Phe Gly Ala Gly Thr Lys Leu Glu Ile Lys
            100                 105

<210>  19
<211>  318
<212>  DNA
<213>  Artificial Sequence

<220>
<223>  Light chain VK1

<220>
<221>  CDS
<222>  (1)..(318)

<400>  19
caa att gtt ctc acc cag tct cca gca acc ctg tct gca tct cca ggg      48
Gln Ile Val Leu Thr Gln Ser Pro Ala Thr Leu Ser Ala Ser Pro Gly
1               5                   10                  15

gag aag gcc gcc atg acc tgc agt gcc agg tca agt gta agt tac ttg      96
Glu Lys Ala Ala Met Thr Cys Ser Ala Arg Ser Ser Val Ser Tyr Leu
            20                  25                  30

tac tgg tac cag cag aag cca ggg tcc tcc ccc aga gcc ctg att tat      144
Tyr Trp Tyr Gln Gln Lys Pro Gly Ser Ser Pro Arg Ala Leu Ile Tyr
        35                  40                  45

gac aca tcc aac ctg gct tct gga gtc cct tct cgc ttc agc ggc agt      192
Asp Thr Ser Asn Leu Ala Ser Gly Val Pro Ser Arg Phe Ser Gly Ser
    50                  55                  60

ggg tct ggg acc gat tac tct ctc aca atc agc agc atg gag gct gaa      240
Gly Ser Gly Thr Asp Tyr Ser Leu Thr Ile Ser Ser Met Glu Ala Glu
65                  70                  75                  80

gat gct gcc act tat tac tgc cag cag tgg agt gat tac ccg ctc acg      288
Asp Ala Ala Thr Tyr Tyr Cys Gln Gln Trp Ser Asp Tyr Pro Leu Thr

85                  90                  95

ttc ggt gct ggg acc aag ctg gag atc aaa                                    318
Phe Gly Ala Gly Thr Lys Leu Glu Ile Lys
            100                 105

<210>  20
<211>  106
<212>  PRT
<213>  Artificial Sequence

<220>
<223>  Synthetic Construct

<400>  20

Gln Ile Val Leu Thr Gln Ser Pro Ala Thr Leu Ser Ala Ser Pro Gly
1               5                   10                  15

Glu Lys Ala Ala Met Thr Cys Ser Ala Arg Ser Ser Val Ser Tyr Leu
            20                  25                  30

Tyr Trp Tyr Gln Gln Lys Pro Gly Ser Ser Pro Arg Ala Leu Ile Tyr
        35                  40                  45

Asp Thr Ser Asn Leu Ala Ser Gly Val Pro Ser Arg Phe Ser Gly Ser
    50                  55                  60

Gly Ser Gly Thr Asp Tyr Ser Leu Thr Ile Ser Ser Met Glu Ala Glu
65                  70                  75                  80

Asp Ala Ala Thr Tyr Tyr Cys Gln Gln Trp Ser Asp Tyr Pro Leu Thr
                85                  90                  95

Phe Gly Ala Gly Thr Lys Leu Glu Ile Lys
            100                 105

<210>  21
<211>  7
<212>  PRT
<213>  Artificial

<220>
<223>  cyclic peptide

<400>  21

Cys Asn Ser Asn Gln Ile Cys
1               5

## Description

The present invention relates to a binding molecule specifically binding to human CD4 for use in the prevention, amelioration and/or treatment of at least symptom of a disease associated with inflammation of the respiratory tract and/or immunologically induced injury of the respiratory tract in a human subject, wherein the binding molecule is administered intranasally and/or via inhalation and/or systemically, as well as to pharmaceutical compositions, pulmonary drug delivery kits and pharmaceutical packages related thereto.

On 11 March 2020, the World Health Organization classified the spread of the novel coronavirus SARS-CoV-2 and the resulting disease COVID-19 as a pandemic (from: https://www.coronavirus.sachsen.de/download/AllgV-Corona-Ausgangsbeschraenkungen_22932929.pdf). The spread of this virus represents a very dynamic and represents a serious burden on the health care system (from: https://www.coronavirus.sachsen.de/download/AllgV-Corona-Ausgangsbeschraenkungen_22932929.pdf). A further strong increase in the number of cases is to be expected (from: https://www.coronavirus.sachsen.de/download/AllgV-Corona-Ausgangsbeschraenkungen_22932929.pdf). Accordingly, the number of seriously ill patients requiring intensive medical care will also increase. The risk to the health of the German population is currently estimated to be high by the Robert Koch Institute (from: https://www.coronavirus.sachsen.de/download/AllgV-Corona-Ausgangsbeschraenkungen_22932929.pdf). Elderly people and people with preexisting underlying diseases are particularly affected by severe disease progression and can die from the disease without the necessary treatment, especially from acute respiratory distress syndrome (ARDS) (from: https://www.coronavirus.sachsen.de/download/AllgV-Corona-Ausgangsbeschraenkungen_22932929.pdf).

ARDS is characterized by acute, severe respiratory distress, oxygen deficiency (hypoxemia) and bilateral diffuse lung infiltrates in the lungs (from: https://viamedici.thieme.de/lernmodule/innere+medizin/acute+respiratory+distress +syndrome+ards). ARDS is caused by diffuse lung damage, which can occur as part of a number of diseases (from: https://viamedici.thieme.de/lernmodule/innere+medizin/acute+respiratory+distress +syndrome+ards). Pathophysiological, the underlying disease leads to a massive release of inflammatory mediators and activation of the coagulation system, which in turn leads to damage of the alveolar membrane and the formation of microthrombi (from: https://viamedici.thieme.de/lernmodule/innere+medizin/acute+respiratory+distress +syndrome+ards). Atelectasis and interstitial/alveolar pulmonary edema develop, leading to respiratory insufficiency, which often requires oxygen therapy or invasive mechanical ventilation (from: https://viamedici.thieme.de/lernmodule/innere+medizin/acute+respiratory+distress +syndrome+ards).

The use of anti-inflammatory drugs is often ineffective and associated with numerous side effects (https://www.the lancet.com/journals/lancet/article/PIIS0140-6736(29)30937-5/fulltext).

Varga et al. (Lancet, 2020; 395(10234): 1417-1418) were able to demonstrate the involvement of endothelial cells from different organs in a number of patients with COVID-19. In addition, they showed the autopsy results of some patients in the supplementary appendix and describe histological signs of ARDS and lymphocytic endotheliitis in lung, heart, kidney and liver with massive centrilobular and parenchyma necrosis. The authors demonstrated the presence of viral elements within the endothelial cells and an accumulation of inflammatory cells. They suggest that SARS-CoV-2 infection facilitates the induction of endotheliitis in multiple organs as a direct result of viral involvement and the host's inflammatory response. In addition, they hypothesize that therapies to stabilize the endothelium while combating viral replication, especially with anti-inflammatory agents, may therefore be potential therapeutic approaches for vulnerable patients with preexisting endothelial dysfunction (Varga et al., supra).

Risk factors include male sex, smoking, hypertension, diabetes, obesity and preexisting cardiovascular disease; all of which are associated with an unfavorable prognosis for COVID-19 (Varga et al., supra).

Currently, there is neither an approved vaccine nor a specific therapy available for the treatment of COVID-19 and SARS-CoV-2-caused ARDS. In particular, no effective pharmacological therapies are available for the treatment of ARDS caused by other entities or disorders such as infections by pathogens, lung diseases, etc..

ARDS is mainly treated symptomatically by mechanical ventilation, fluid management or extracorporeal membrane oxygenation (ECMO) (Lewandowski K., Crit Care. 2000; 4(3): 156-168). Pharmacological therapies, such as immunosuppressive drugs (i.e. immunosuppressants) often have to be administered systemically, exhibit significant organ toxicity and permanently deteriorate the function of the immune system.

Preliminary results with small case numbers have been published in China for the treatment of the cytokine storm by COVID-19 with tocilizumab (RoActemra^{®}; a recombinant humanized anti-human IL-6 receptor monoclonal antibody) (Xu X. et al., Proc Natl Acad Sci USA, 2020, May 19;117(20):10970-10975). However, this study is merely a retrospective analysis.

In addition, all previous treatment methods have a systemic administration route in common.

Accordingly, there is a strong medical need for the therapeutic approaches which allow the prevention, amelioration and/or treatment of at least symptom of a disease associated with inflammation of the respiratory tract and/or immunologically induced injury of the respiratory tract in a human subject, in particular for the prevention, amelioration and/or treatment of ARDS symptoms. Further, there is a strong medical need for the therapeutic approaches which allow the prevention, amelioration and/or treatment of at least symptom of SARS-CoV-2 in human patients, and in particular for the prevention, amelioration and/or treatment of ARDS caused by SARS-CoV-2.

The present invention solves these problems.

In one embodiment, the present invention relates to a binding molecule specifically binding to human CD4 for use in the prevention, amelioration and/or treatment of at least symptom of a disease associated with inflammation of the respiratory tract and/or immunologically induced injury of the respiratory tract in a human subject, wherein the binding molecule is administered intranasally and/or via inhalation and/or systemically.

It is surprisingly found that the topical, local administration of a human CD4-specific binding molecule via inhalation or intranasal administration results in the amelioration of symptoms of diseases associated with inflammation of the respiratory tract and immunologically induced injury of the respiratory tract. Accordingly, the administration of such binding molecules specific for human CD4 are suitable for the prevention, amelioration and treatment of these diseases. Alternatively, the human CD4-specific binding molecule may be administered systemically. This alternative is in particular advantageous for subjects suffering from severe, life-threatening conditions, such as severe ARDS.

Without being bound to the theory, is hypothesized that, by blocking CD4 molecules on T-helper cells and macrophages, local immune reactions are attenuated, which ultimately leads to an improvement of the respiratory situation and thus survival of human subjects suffering from respiratory diseases associated with inflammation of the respiratory tract and/or immunologically induced injury of the respiratory tract. In particular, the administration of a binding molecule specifically binding to human CD4, such as an anti-CD4 antibody, via inhalation and/or intranasal administration and/or systemically, is suitable for the treatment and amelioration of ARDS, especially induced or caused by SARS-COV-2.

In order to avoid side effects and/or exert a local effect effectively, the administration of a binding molecule specifically binding to human CD4, such as an anti-CD4 antibody, via inhalation and/or intranasal administration is preferred.

In particular, chimeric antibody CD4.16H5.chimIgG4 produced under GMP conditions is particularly suitable herein in terms of the epitope bound on human CD4, low antigenicity and isotype (human IgG₄).

Moreover, promising preclinical data are available for CD4.16H5.chimlgG4 or MAX.16H5 IgG1 (which has the same variable regions as CD4.16H5.chimlgG4) in humanized mouse models for the suppression of an allogeneically induced inflammation (GvHD). Proof-of-concept studies have demonstrated the efficacy of a chimeric anti-human CD4 antibody CD4.16H5.chimlgG4 or MAX.16H5 IgG1 in preventing GvHD (i.e. an allogeneically induced inflammation) while maintaining the anti-tumor effect (Kohlhaw K. et al., Transplant. Proc. 2001 33:2371-2373; Laub R. et al., J. Immunol. Methods 2000 246:37-50; Laub R. et al., J. Immunol. 2002, 169: 2947; Schmidt F. et al.., Cytometry A 2015; 87:334-45; Hilger N. et al., Front Immunol. 2018 Oct 22; 9:2408, and Stahl L. et al., Front Immunol. 2019; 10:1035).

The present invention is particularly suitable for the treatment or amelioration of respiratory symptoms of COVID-19 patients requiring mechanical ventilation, due to the advantageous immunomodulatory and in particular immunosuppressive effects of the binding molecules herein for use of the invention by inhalation and targeted delivery to the lungs. Herein, the use of CD4.16H5.chimlgG4 is particularly preferred.

The administration via inhalation or intranasal administration provides for a favorable side effect profile as compared to systemic immunosuppression with conventional immunosuppressants or systemically administered antibodies, such as tocilizumab. In particular, a favorable side effect profile is expected as no immunosuppression maintained over a longer period of time is required, which negatively affects the immunological defense system as a whole.

Alternatively, the human CD4-specific binding molecule may be administered systemically. This alternative is in particular advantageous for subjects suffering from severe, life-threatening conditions, such as severe ARDS.

Preferably, the disease associated with inflammation of the respiratory tract and/or immunologically induced injury of the respiratory tract is selected from Acute Respiratory Distress Syndrome (ARDS), asthma, chronic obstructive pulmonary disease (COPD), lung fibrosis pulmonary Graft-versus-Host-Disease (GvHD), aspiration (inhalation) of food components into the lungs, burns, pregnancy complications, in particular selected from amniotic fluid embolism, pre-eclampsia, infection of uterine tissue before, during or after a miscarriage (septic abortion), breast injury, in particular lung contusion, coronary artery bypass surgery, drowning, inflammation of the pancreas (pancreatitis), inhalation of large quantities of smoke, inhalation of other toxic gases, damage to the lungs through inhalation of highly concentrated oxygen, life threatening or serious injuries, overdose of substances in particular selected from heroin, methadone, propoxyphene or acetylsalicylic acid, pneumonia, prolonged or threateningly low blood pressure (circulatory shock), pulmonary embolism, severe, systemic infection (sepsis), stroke or cramping seizure, transfusions of more than about 15 blood preserves in a short time, infection or sepsis with extra-pulmonary focus, disruption of microcirculation (shock), systemic inflammatory response syndrome (SIRS), extrapulmonary organ failure, and metabolic derailment. For example, systemic inflammatory response syndrome (SIRS) may be caused by sterile inflammation, extensive crush injuries, polytrauma, disseminated intravascular coagulation, drugs and toxins (such as salicylic acid, tricyclic antidepressants, bleomycin, organic phosphates, etc.). For example, extrapulmonary organ failure may be kidney failure or liver failure. For example, metabolic derailment may be diabetic ketoacidosis.

These diseases are characterized by local inflammation of cells and tissues of the lower respiratory tract, in particular the lungs. The delivery of the human CD4-specific binding molecules specifically to the lungs is expected to exhibit an immunosuppressive and immunomodulatory effect specifically at the target site.

Chronic obstructive pulmonary disease (COPD) is an obstructive lung disease characterized by long-term breathing problems and poor airflow (see e.g. https://en.wikipedia.org/wiki/Chronic_obstructive_pulmonary_disease, entry of August 2020). The main symptoms include shortness of breath and cough with sputum production. The diagnosis is based on poor airflow as measured by lung function tests. In contrast to asthma, the airflow reduction does not improve much with the use of a bronchodilator. COPD develops as a significant and chronic inflammatory response to inhaled irritants. Chronic bacterial infections can also add to this inflammatory state. The inflammatory cells involved include neutrophil granulocytes and macrophages. Smoking patient may additionally have Tc1 lymphocyte involvement and some patients with COPD have eosinophil involvement.

Asthma is a common long-term inflammatory disease of the airways of the lungs (see e.g. https://en.wikipedia.org/wiki/Asthma, entry of August 2020). It is characterized by variable and recurring symptoms, reversible airflow obstruction, and easily triggered bronchospasms. Symptoms include episodes of wheezing, coughing, chest tightness, and shortness of breath. Asthma is the result of chronic inflammation of the conducting zone of the airways, in particular the bronchi and bronchioles, which subsequently results in increased contractability of the surrounding smooth muscles. Typical changes in the airways include an increase in eosinophils and thickening of the lamina reticularis. Involved inflammatory cells include T lymphocytes, macrophages, and neutrophils. Further components of the immune system involved in asthma include cytokines, chemokines, histamine, and leukotrienes, among others.

Pulmonary graft versus host disease (GvHD) is a thoracic manifestation that can complicate hematopoietic stem cell transplantation (see e.g. https://radiopaedia.org/articles/graft-versus-host-disease-pulmonary-manifestations, entry of August 2020). Pulmonary GvHD can be divided into acute and chronic disease. Acute pulmonary GvHD involvement is rare, the median time of onset of respiratory symptoms is around five months and reported radiological features include mild perihilar or diffuse interstitial fibrosis, pulmonary cysts and pulmonary nodules. Chronic pulmonary GvHD usually presents as a bronchiolitis obliterans and/or organizing pneumonia. Moreover, pulmonary fibrosis may occur.

Lung fibrosis, also termed pulmonary fibrosis, is a condition in which the lungs become scarred over time (see e.g. https://www.mayoclinic.org/diseases-conditions/pulmonary-fibrosis/symptoms-causes/syc-20353690; entry of August 2020). Symptoms include shortness of breath, a dry cough, feeling tired, weight loss, and nail clubbing. Complications may include pulmonary hypertension, respiratory failure, pneumothorax, and lung cancer. Pulmonary fibrosis involves gradual exchange of normal lung parenchyma with fibrotic tissue. The replacement of normal lung with scar tissue causes irreversible decrease in oxygen diffusion capacity, and the resulting stiffness or decreased compliance makes pulmonary fibrosis a restrictive lung disease.

ARDS is characterized by acute, severe respiratory distress, oxygen deficiency (hypoxemia) and bilateral diffuse lung infiltrates in the lungs. ARDS is caused by diffuse lung damage, which can occur as part of a number of diseases. Pathophysiological, the underlying disease leads to a massive release of inflammatory mediators and activation of the coagulation system, which in turn leads to damage of the alveolar membrane and the formation of microthrombi. Atelectasis and interstitial/alveolar pulmonary edema develop, leading to respiratory insufficiency, which often requires oxygen therapy or invasive mechanical ventilation.

Diagnostic criteria for ARDS have changed over time as understanding of the pathophysiology has evolved. The international consensus criteria for ARDS were most recently updated in 2012 and are known as the "Berlin definition". According to the 2012 Berlin definition, ARDS is characterized by the following:
- lung injury of acute onset, within 1 week of an apparent clinical insult and with progression of respiratory symptoms
- bilateral opacities on chest imaging (such as chest radiograph or CT) not explained by other lung pathology (e.g. effusion, lobar/lung collapse, or nodules),
- respiratory failure not explained by heart failure or volume overload,
- decreased PaO₂/FiO₂ ratio
decreased PaO₂/FiO₂ ratio indicates reduced arterial oxygenation from the available inhaled gas; PaO₂/FiO₂ ratio is the ratio of partial pressure arterial oxygen and fraction of inspired oxygen, known as the Horowitz index or Carrico index.

A decreased PaO₂/FiO₂ ratio is further classified as follows:
mild ARDS: 201 - 300 mmHg (≤ 39.9 kPa)
moderate ARDS: 101 - 200 mmHg (≤ 26.6 kPa)
severe ARDS: ≤ 100 mmHg (≤ 13.3 kPa)

Radiologic, in particular CT analysis and ultrasound analysis can be used in diagnosis of ARDS. Generally, radiographic findings of fluid accumulation (i.e. pulmonary edema) affecting both lungs and unrelated to increased cardiopulmonary vascular pressure (such as in heart failure) may be indicative of ARDS.

Ultrasound findings that may be indicative ARDS include the following:
Anterior subpleural consolidations
Absence or reduction of lung sliding
"Spared areas" of normal parenchyma
Pleural line abnormalities (irregular thickened fragmented pleural line) Nonhomogeneous distribution of B-lines (a characteristic ultrasound finding indicative of fluid accumulation in the lungs).

As shown in the example, binding molecules specifically binding to human CD4 are particularly suitable for treating symptoms of ARDS by administration via inhalation or intranasal administration. Alternatively, the human CD4-specific binding molecule may be administered systemically. For example, by blocking CD4 molecules on T-helper cells and macrophages, local immune reactions are attenuated, which ultimately leads to an improvement of the respiratory situation and thus survival of human subjects suffering from ARDS.

Accordingly, the use in the prevention, amelioration and/or treatment of at least symptom of ARDS is particularly preferred. The ARDS may have any cause. Known causes of ARDS include infection by a pathogen, in particular SARS-CoV-2, pneumonia, aspiration, inhalational lung injury, lung contusion, chest trauma, near-drowning, sepsis, shock, pancreatitis, trauma, fat embolism, cardiopulmonary bypass, Transfusion-related acute lung injury (TRALI), burns, or increased intracranial pressure. Pathogens as cause of ARDS include e.g. viruses, bacteria, protozoa and fungi, in particular viruses and bacteria. Among viruses causing ARDS, for example coronaviruses, such as SARS-CoV-1, MERS-CoV or SARS-CoV-2, influenza viruses, Herpes simplex viruses and CMV are known examples. Such viruses are preferred causes of ARDS. ARDS caused by viruses is also termed "virus-induced ARDS". Bacteria causing ARDS are also known and include, for example, *Staphylococcus aureus* and *Streptococcus pneumoniae.*

Coronaviruses have caused large-scale pandemics in the past decades, including SARS and Middle East respiratory syndrome (MERS) (Drosten, C. et al.: Identification of a novel coronavirus in patients with severe acute respiratory syndrome. N. Engl. J. Med. 348, 1967-1976 (2003); Zaki, A. M. et al.: Isolation of a novel coronavirus from a man with pneumonia in Saudi Arabia. N. Engl. J. Med. 367, 1814-1820 (2012)).

The coronavirus is preferably selected from the group consisting of severe acute respiratory syndrome coronavirus 2 (SARS-CoV-2), severe acute respiratory syndrome coronavirus 1 (SARS-CoV-1), Middle East respiratory syndrome coronavirus (MERS-CoV), human coronavirus NL63 (HCoV NL63), human coronavirus OC43 (HCoV OC43), human coronavirus HLU1 (HCoV HKU1), and human coronavirus 229E (HCoV 229E).

An overview on Human Coronavirus-229E, -OC43, -NL63, and -HKU1 is for example provided in Liu, Ding X. et al. ("Human Coronavirus-229E, -OC43, -NL63, and -HKU1." Reference Module in Life Sciences (2020): B978-0-12-809633-8.21501-X. doi:10.1016/B978-0-12-809633-8.21501-X).

Accordingly, it is preferred that the ARDS is caused by a pathogen, in particular SARS-CoV-2, pneumonia, aspiration, inhalational lung injury, lung contusion, chest trauma, near-drowning, sepsis, shock, pancreatitis, trauma, fat embolism, cardiopulmonary bypass, Transfusion-related acute lung injury (TRALI), burns, or increased intracranial pressure.

As demonstrated in the example, it is preferred that the ARDS is caused by a pathogen, more preferably wherein the ARDS is caused by a pathogen selected from a virus and a bacteria, even more preferably by a virus. It is particularly preferred that the virus causing ARDS is selected from a coronavirus, such as SARS-CoV-1, MERS-CoV or SARS-CoV-2, an influenza virus, a Herpes simplex virus and a CMV. The use according to the prevention for preventing, ameliorating and/or treating at least one symptom caused by SARS-CoV-2 is particularly preferred.

Accordingly, in a preferred embodiment, a binding molecule for use of the invention is preferred, wherein the ARDS is caused by SARS-CoV-2.

Accordingly, in another preferred embodiment, the binding molecule for use of the invention is preferred, wherein the human subject is diagnosed to have a SARS-CoV-2 infection. Methods for the diagnosis of an SARS-CoV-2 infection are known in the art and include *in vitro* tests detecting SARS-CoV-2-specific nucleic acids in a sample obtained from the subject, or detecting antibodies, for example IgM- or IgG-antibodies, specific for SARS-CoV-2 in a sample obtained from the subject. In *vitro* tests and methods for detecting SARS-CoV-2-specific nucleic acids in a sample obtained from the subject are known in the art and may for example employ PCR-based methods, in particular rRT-PCR methods detecting viral RNA.

The sample obtained from the subject may for example be a pharyngeal swab, blood, sputum, feces, urine, nasal sample, bronchoalveolar lavage fluid or a fibrobronchoscope brush biopsy.

Accordingly, in another preferred embodiment, the human subject suffers from SARS-CoV-2.

The term "suffers from SARS-CoV-2" or "suffering from SARS-CoV-2" as used herein may be understood in the broadest sense in a way that the subject has developed a pathological condition associated with SARS-CoV-2. The patient suffering from a disorder or disease not necessarily but optionally bears medicinal symptoms.

A human subject suffering from SARS-CoV-2 preferably has detectable amounts of SARS-CoV-2 in a sample obtained from the subject or had detectable amounts of SARS-CoV-2 in a sample obtained from the subject about 1 day to 6 months before the sample has been obtained. It is preferred that the subject exhibits at least one symptom of an SARS-CoV-2 infection.

Symptoms of SARS-CoV-2 include fever, chills, coughing, shortness of breath and/or difficulty breathing, including ARDS, fatigue, muscle and/or body aches, headache, new loss of taste and/or smell, sore throat, congestion and/or runny nose, nausea and/or vomiting, and diarrhea.

More generally, it is preferred that the ARDS is caused by a pathogen, in particular a virus or a bacterium, and/or wherein the human subject is diagnosed to have a pathogen infection, in particular wherein the pathogen is a virus or a bacterium, and/or wherein the human subject suffers from a pathogen infection, in particular in particular wherein the pathogen is virus or a bacterium. In a more preferred embodiment, the virus is selected from a coronavirus, such as SARS-CoV-1, MERS-CoV or SARS-CoV-2, an influenza virus, a Herpes simplex virus and a CMV.

In one preferred embodiment, the binding molecule specifically binding to human CD4 is for use in the amelioration and/or treatment of at least symptom of Acute Respiratory Distress Syndrome (ARDS). It is preferred that the symptoms include one or more of acute, severe respiratory distress, oxygen deficiency (hypoxemia) and bilateral diffuse lung infiltrates. For example, an amelioration may include an increase in PaO₂/FiO₂ ratio as compared the start of treatment, for example by 1%, 2%, 5%, 10%, 20%, 30%, 40%, 50%, 100%, 200%, 300% or more, or until normal PaO₂/FiO₂ ratio is reached, preferably not exceeding such normal values. For example, an amelioration may include a decrease in bilateral diffuse lung infiltrates as compared the start of treatment. Such decrease in bilateral diffuse lung infiltrates may for example be detected by *in vivo* imaging, in particular by using X-rays, such as CT, or ultrasound imaging. Moreover, histopathological detection of inflammatory cells such as macrophages in a biopsy is possible. A decrease in bilateral diffuse lung infiltrates may be determined quantitatively or semi-quantitatively, e.g. by visual inspection.

Hypoxemia is a condition characterized by an abnormally low level of oxygen in the blood, preferably in arterial blood (see e.g. https://en.wikipedia.org/wiki/Hypoxemia; entry of August 2020).

In a further more preferred embodiment, the human subject suffers from ARDS.

It is expected these subject will in particular benefit from the treatment with the binding molecules specifically binding to human CD4 by intrasal administration or administration via inhalation and/or systemic administration.

Further, it is further hypothesized that a subject will in particular benefit from the treatment with the binding molecules herein which exhibits preexisting endotheliitis and/or preexisting endothelial dysfunction, in particular wherein the preexisting endotheliitis and/or preexisting endothelial dysfunction is in the respiratory tract, in particular the lungs. By intrasal administration or administration via inhalation, the binding molecules specifically binding to human CD4 will exert a local, beneficial immunosuppressive and immunomodulatory effect. Thereby, the endotheliitis and/or preexisting endothelial dysfunction of the respiratory tract, in particular the lungs, will be reduced or reversed. Alternatively, the human CD4-specific binding molecule may be administered systemically. This alternative is in particular advantageous for subjects suffering from severe, life-threatening conditions, such as severe ARDS.

Endotheliitis is an immune response within the endothelium in blood vessels, in which the blood vessels become inflamed(from: https://en.wikipedia.org/wiki/Endotheliitis; entry of August 2020). The condition can cause edema of the surrounding tissue, including the stroma.

Accordingly, in a further more preferred embodiment, the human subject exhibits preexisting endotheliitis and/or preexisting endothelial dysfunction, in particular of the respiratory tract, in particular the lungs.

A preexisting endotheliitis and/or preexisting endothelial dysfunction is understood as endotheliitis and/or endothelial dysfunction which already occurred and persisted prior to the disease associated with inflammation of the respiratory tract and/or immunologically induced injury of the respiratory tract.

The respiratory tract is the subdivision of the respiratory system involved with the process of respiration in mammals. The respiratory tract is lined with respiratory mucosa or respiratory epithelium (see e.g. https://en.wikipedia.org/wiki/Respiratory_tract; entry of August 2020). The respiratory tract includes the upper airways, including the oropharynx and larynx, followed by the lower airways, which include the trachea followed by bifurcations into the bronchi and bronchioli. The upper and lower airways are called the conductive airways. The terminal bronchioli then divide into respiratory bronchioli which then lead to the ultimate respiratory zone, the alveoli, or deep lung. The lungs are part of the lower respiratory tract or, lower airways or lower respiratory airways.

It is preferred the disease associated with inflammation of the respiratory tract and/or immunologically induced injury of the respiratory tract is a disease associated with inflammation of the lower airways and/or immunologically induced injury of the lower airways. Also, in another preferred embodiment, the lower airways are a preferred portion of the respiratory tract. For example, endotheliitis and/or preexisting endothelial dysfunction of the respiratory tract is preferably endotheliitis and/or preexisting endothelial dysfunction of the lower airways.

In a more preferred embodiment, the disease associated with inflammation of the respiratory tract and/or immunologically induced injury of the respiratory tract is a disease associated with inflammation of the lungs and/or immunologically induced injury of the lungs. Also, in another preferred embodiment, the lungs are a preferred portion of the respiratory tract. For example, endotheliitis and/or preexisting endothelial dysfunction of the respiratory tract is preferably endotheliitis and/or preexisting endothelial dysfunction of the lungs.

Moreover, several risk factors have been identified for developing ARDS, in particular ARDS caused by SARS-CoV-2 (see e.g. Varga et al. supra). These risk factors include, for example, male sex, age of 60 years or more, smoking, elevated blood pressure, diabetes, obesity and a cardiovascular disease (see e.g. Varga et al. supra).. It is therefore preferred that the binding molecule is administered to a subject exhibiting one or more of the risk factors. Accordingly, in a further more preferred embodiment, the human subject exhibits one or more risk factors selected from male sex, age of 60 years or more, smoking, elevated blood pressure, diabetes, obesity and a cardiovascular disease.

It is surprisingly found that the intranasal administration or administration via inhalation or systemic administration of a binding molecule specifically binding to human CD4 can be used in the prevention, amelioration and/or treatment of at least symptom of a disease associated with inflammation of the respiratory tract and/or immunologically induced injury of the respiratory tract in a human subject.

By blocking CD4 molecules on T-helper cells and macrophages, local immune reactions are attenuated, which ultimately leads to an improvement of the respiratory situation and thus survival of human subjects suffering from such respiratory diseases.

Accordingly, in a preferred embodiment, the administration of the binding molecule decreases mortality.

Accordingly, the chances of survival of the subject are increased and/or the period of survival of the subject is increased, for example by 1 day or more, 7 days or more, 2 or 3 weeks or more, 1 month or more or 1 year or more.

The pulmonary delivery of the binding molecule specifically binding to human CD4, by intranasal administration or via inhalation, ultimately leads to an improvement of the respiratory situation. Alternatively, the human CD4-specific binding molecule may be administered systemically. An improvement of the respiratory situation is in particular advantageous for subjects requiring and/or obtaining mechanical ventilation. Exemplary subjects requiring and/or obtaining mechanical ventilation are subjects suffering from ARDS, such as ARDS caused by a pathogen, in particular by a coronavirus, most preferably by SARS-CoV-2. The administration to the subject can ameliorate and/or treat symptoms of ARDS. It is preferred that the symptoms include one or more of acute, severe respiratory distress, oxygen deficiency (hypoxemia) and bilateral diffuse lung infiltrates, as explained above. Thereby, the duration of mechanical ventilation of the subject can be reduced, as mechanical ventilation may not be required anymore.

Therefore, preferably, the administration of the binding molecule reduces the duration of mechanical ventilation of the subject. For example, the duration of mechanical ventilation of the subject can be reduced by at least 1 day, at least 7 days, or at least 2, 3, 4, 5 or 6 weeks as compared to mechanical ventilation without the administration of the binding molecule. For example, the duration of mechanical ventilation of the subject can be reduced to a duration of 1, 2, 3, 4, 5, 6, or 7 days or less, 2, 3, 4, 5 or 6 weeks or less, or 1, 2 or 3 months or less.

The preferred antibody 16H5.chimlgG4 for use in the invention exhibits beneficial effects with respect to immunomodulation and immunosuppression. Thereby, the binding molecule can exhibit a local, topical immunosuppressive effect by blocking CD4 on T helper cells and macrophages in the lungs. This can be in particular be helpful for subjects with endotheliitis in the respiratory tract and in particular in the lungs.

The vascular endothelium is an active paracrine, endocrine, and autocrine organ that is indispensable for the regulation of vascular tone and the maintenance of vascular homoeostasis (Varga et al., supra). Endothelial dysfunction shifts the vascular equilibrium towards more vasoconstriction with subsequent organ ischemia, inflammation with associated tissue edema, and a pro-coagulant state (Varga et al., supra). Endotheliitis is preferably characterized by the accumulation of inflammatory cells and optionally further by endothelial and inflammatory cell death. The accumulated inflammatory cells are for example lymphocytes and/or macrophages.

Preferably, the administration of the binding molecule specifically modulates immune responses to inflammatory cells in the lungs, in particular wherein the inflammatory cells are T helper cells and/or macrophages. Thereby, local immune reactions in the lungs are suppressed and/or ameliorated.

Accordingly, in a yet further preferred embodiment, the administration of the binding molecule specifically modulates immune responses to inflammatory cells in the respiratory tract, in particular wherein the inflammatory cells are T helper cells and/or macrophages, and/or the administration of the binding molecule ameliorates and/or suppresses local immune reactions in the respiratory tract, and/or the administration of the binding molecule ameliorates and/or suppresses endotheliitis, in particular lymphocytic endotheliitis, in the respiratory tract.

Preferably, the at least symptom of the disease associated with inflammation of the respiratory tract and/or immunologically induced injury of the respiratory tract is selected from acute severe respiratory distress, hypoxemia and bilateral diffuse pulmonary infiltrates.

Acute severe respiratory distress, hypoxemia and bilateral diffuse pulmonary infiltrates are for example observed in subjects suffering from ARDS. The administration of the binding molecules for use in the invention via inhalation or by intranasal administration or by systemic administration is suitable for ameliorating the symptoms.

Accordingly, in a yet further embodiment, the treatment herein ameliorates or treats at least symptom selected from acute severe respiratory distress, hypoxemia and bilateral diffuse lung infiltrates.

In the uses of the present invention, a binding molecule specifically binding to human CD4 is administered to a subject.

A binding molecule is understood as chemical molecule, preferably a polypeptide, a peptide or small molecule, which exhibits specific binding to the target or antigen indicated. According to the present invention, the target or antigen is the protein human CD4. Therefore, binding molecules for use of the invention specifically bind to human CD4. Preferably, the binding molecule is an immunoglobulin comprising molecule, i.e. comprises at least one Ig domain or an anti-human CD4 antibody.

"Specific binding" is understood that the binding of the binding molecule to human CD4 is at least 50-fold, preferably at least 100-fold stronger than the binding to a control protein such as albumin, as determined e.g. by Western Blot analysis or ELISA.

It is preferred that the binding molecule specifically binding to human CD4 is selected from an anti-human CD4 antibody, a human CD4 ligand or a peptide.

The term "anti-human CD4 antibody", as used herein, means any polypeptide which has structural similarity to a naturally occurring antibody and is capable of binding to human CD4, wherein the binding specificity is determined by the CDRs of the polypeptides. Hence, "anti-human CD4 antibody" is intended to relate to an immunoglobulin-derived structure with binding to human CD4. The antigen-binding fragment is understood as polypeptide which comprises at least one antigen binding fragment of a full-length antibody. Antigen binding fragments consist of at least the variable domain of the heavy chain and the variable domain of the light chain, arranged in a manner that both domains together are able to bind to the specific antigen.

Preferably, the binding molecule specifically binding to human CD4 is an anti-CD4 antibody. Suitable Anti-human CD4 antibodies are generally well-known in the art. Numerous anti-human CD4 antibodies are commercially available.

The term "antigen" is used herein to refer to a substance that is capable of specifically combining, interacting or otherwise associating with said binding molecule or antibody. In the context of the anti-human CD4 antibody for use of the present invention, the antigen is meant to be human CD4. In the context of the anti-human CD4 antibody for use of the present invention, the antigen is human CD4.

According to the invention, the term "anti-human CD4 antibody" refers to an antibody, which has the ability to specifically bind to human CD4.

"CD4" or "cluster of differentiation 4" refers to a protein, more precisely a surface glycoprotein, well known to the person skilled in the art (cf. Bowers et al., 1997). In the present context, CD4 may also refer to a fragment of full-length CD4, or an otherwise modified form of CD4, provided that the fragment or otherwise modified form still functions as an antigen in the context of the antibody or binding molecule for use of the present invention. Human CD4 is well known in the art. For example, the mRNA sequence is disclosed as sequence entry NM_000616 (version of July 12, 2020). For example, the protein sequence is disclosed as UniProt entry P01730, version last modified on June 17, 2020. CD4 is a member of the immunoglobulin superfamily. It has four immunoglobulin domains designated D1 to D4, that are exposed on the extracellular surface of the cell: D1 and D3 resemble immunoglobulin variable (IgV) domains. D2 and D4 resemble immunoglobulin constant (IgC) domains.

Preferably, the binding molecule for use herein binds to an epitope in the extracellular part of human CD4. Preferably, the binding molecule binds to an epitope in immunoglobulin domain D1, immunoglobulin domain D2, immunoglobulin domain D3 or immunoglobulin domain D4. Techniques for determining the epitope bound are known in the art and include e.g. alanine scanning.

For the development as a therapeutic antibody, 16H5.chimIgG4 and MAX.16H5 IgG1, respectively, are advantageous in view of their high affinity to CD4 (Stahl L. et al, supra). It was shown that 16H5.chimlgG4 and MAX.16H5 IgG1 share some fine specificities with gp120 with regard to the recognition of different mutated CD4 versions. The peptide T_{b}YIC_{b}E_{b}VEDQK_{Ac}EE was reported to inhibit CD4 binding of both gp120 and MAX.16H5 IgG1 (or 16H5.chimlgG4).

As used herein, the terms "antibody" and "antibodies" include full length antibodies, antigen-binding fragments of full length antibodies, and molecules comprising antibody CDRs, VH regions or VL regions. Examples of antibodies include monoclonal antibodies, recombinantly produced antibodies, monospecific antibodies, multispecific antibodies (including bispecific antibodies), human antibodies, humanized antibodies, chimeric antibodies, primatized antibodies, immunoglobulins, synthetic antibodies, tetrameric antibodies comprising two heavy chain and two light chain molecules, an antibody light chain monomer, an antibody heavy chain monomer, an antibody light chain dimer, an antibody heavy chain dimer, an antibody light chain-antibody heavy chain pair, intrabodies, heteroconjugate antibodies, antibody-drug conjugates, single domain antibodies, monovalent antibodies, single chain antibodies or single-chain Fvs (scFv), camelized antibodies, affybodies, Fab fragments, F(ab')₂ fragments, disulfide-linked Fvs (sdFv), anti-idiotypic (anti-Id) antibodies (including, e.g., anti-anti-Id antibodies), and antigen-binding fragments of any of the above. Also, antibodies described herein may refer to polyclonal antibody populations. Antibodies can be of any type (e.g., IgG, IgE, IgM, IgD, IgA or IgY), any class (e.g., IgG₁, IgG₂, IgG₃, IgG₄, IgA₁ or IgA₂), or any subclass (e.g., IgG₂ₐ or IgG_{2b}) of immunoglobulin molecule. It is preferred that antibodies used herein are IgG antibodies, or a class (e.g., human IgG₁ or IgG₄) or subclass thereof.

It is preferred that the antibody for use herein has an Fc portion of human IgG₁ or human IgG₄ isotype, more preferably of human IgG₄ isotype. Preferably, CD4.16H5.chimlgG4 or MAX.16H5 IgG1 may be used, in particular CD4.16H5.chimlgG4.

It is preferred that the antibody for use herein is a chimeric antibody which has an Fc portion of human IgG₁ or human IgG₄ isotype, more preferably of human IgG₄ isotype. In particular, CD4.16H5.chimIgG4 may be used.

As the binding molecule is administered to a subject, which is preferably a human, it is preferred that the antibodies exhibit reduced antigenicity in the subject.

Accordingly, it is preferred that the binding molecule specifically binding to human CD4 is a monoclonal antibody or an antigen-binding fragment thereof, or is a human, humanized, primatized or a chimeric antibody.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and disclosed, for example, in Harlow E & Lane D, Antibodies: A Laboratory Manual, (Cold Spring Harbor Laboratory Press, 2nd ed. 1988); Hammerling GJ et al., in: Monoclonal Antibodies and T-Cell Hybridomas 563 681 (Elsevier, N.Y., 1981).

Antigen-binding fragments of antibodies include antibody fragments which specifically bind to human CD4 and can be generated by any technique known to those of skill in the art. For example, Fab and F(ab')2 fragments can be produced by proteolytic cleavage of immunoglobulin molecules, using enzymes such as papain (to produce Fab fragments) or pepsin (to produce F(ab')2 fragments). For example, antigen-binding fragments may be used which comprise the variable regions of an anti-human CD4 antibody.

Methods for producing human antibodies are also known in the art. Examples of mice capable of producing human antibodies include the Xenomouse^{™} (Abgenix, Inc.; US 6,075,181 and US 6,150,184), and the HuAb-Mouse^{™} (Mederex, Inc./Gen Pharm; US 5,545,806 and US 5,569, 825). Human antibodies which specifically bind to human CD4 can be made by a variety of methods known in the art including phage display methods using antibody libraries derived from human immunoglobulin sequences (see e.g. US 4,444,887, US 4,716,111, and US US 5,885,793; WO 98/46645, WO 98/50433, WO 98/24893, WO 98/16654, WO 96/34096, WO 96/33735, and WO 91/10741). Also, human antibodies can be produced using mouse-human hybridomas.

A humanized antibody is capable of specific binding to a predetermined antigen and which comprises a framework region having substantially the amino acid sequence of a human immunoglobulin and CDRs having substantially the amino acid sequence of a non-human immunoglobulin, such as a murine, rat or rabbit immunoglobulin. A humanized antibody can also comprise at least a portion of an immunoglobulin constant region (Fc), typically that of a human immunoglobulin. The antibody also can include the CH1, hinge, CH2, CH3, and CH4 regions of the heavy chain. A humanized antibody can be selected from any class of immunoglobulins, including IgM, IgG, IgD, IgA and IgE, and any isotype, including IgG1, IgG₂, IgG3 and IgG4. Humanized antibodies can be produced using a variety of techniques known in the art, including, CDR-grafting (EP 239400; WO 91/09967; US 5,225,539, US 5,530,101, and US 5,585,089), veneering or resurfacing (EP 592106 and EP 519596; Padlan EA (1991) Mol Immunol 28(4/5): 489-498; Studnicka GM et al., (1994) Prot Engineering 7(6): 805-814; and Roguska MA et al., (1994) PNAS 91: 969-973), chain shuffling (US 5,565,332), and techniques disclosed in, e.g., US. 6,407,213, US 5,766,886, WO 93/17105; Tan P et al., (2002) J Immunol 169: 1119-25; Caldas C et al., (2000) Protein Eng. 13(5): 353-60; Morea V et al., (2000) Methods 20(3): 267-79; Baca M et al., (1997) J Biol Chem 272(16): 10678-84; Roguska MA et al., (1996) Protein Eng 9(10): 895 904; Couto JR et al., (1995) Cancer Res. 55 (23 Supp): 5973s-5977s; Couto JR et al., (1995) Cancer Res 55(8): 1717-22; Sandhu JS (1994) Gene 150(2): 409-10 and Pedersen JT et al., (1994) J Mol Biol 235(3): 959-73).

A chimeric antibody is a molecule in which different portions of the antibody are derived from different immunoglobulin molecules. For example, a chimeric antibody can contain a variable region of a mouse or rat monoclonal antibody fused to a constant region of a human antibody. Methods for producing chimeric antibodies are known in the art (for example in Morrison SL (1985) Science 229: 1202-7; Oi VT & Morrison SL (1986) BioTechniques 4: 214-221; Gillies SD et al., (1989) J Immunol Methods 125: 191-202; US 5,807,715, US 4,816,567, US 4,816,397, and US 6,331,415).

An example of a primatized anti-human CD4 antibody which can be used according to the invention is Clenoliximab (WHO INN).

Moreover, it is advantageous if the binding molecule does not deplete CD4-expressing cells and/or mediate stripping of CD4 from lymphocyte cell surface *in vivo.*

Accordingly, it is preferred that the binding molecule specifically binding to human CD4 is a non-depleting anti-human CD4 antibody.

As used herein, the terms "VH region" and "VL region" refer to single antibody heavy and light chain variable regions, respectively, comprising FR (Framework Regions) 1, 2, 3 and 4 and CDR (Complementarity Determining Regions) 1, 2 and 3 (see Kabat et al., (1991) Sequences of Proteins of Immunological Interest (NIH Publication No. 91-3242, Bethesda).

As used herein, the term "CDR" or "complementarity determining region" means the noncontiguous antigen combining sites found within the variable region of both heavy and light chain polypeptides. These particular regions have been described by Kabat et al., J. Biol. Chem. 252, 6609-6616 (1977) and Kabat et al., Sequences of protein of immunological interest. (1991), by Chothia et al., J. Mol. Biol. 196:901-917 (1987), and by MacCallum et al., J. Mol. Biol. 262:732-745 (1996), where the definitions include overlapping or subsets of amino acid residues when compared against each other. According to one preferred alternative of the invention, the term "CDR" is a CDR as defined by MacCallum et al., J. Mol. Biol. 262:732-745 (1996) and Martin A. "Protein Sequence and Structure Analysis of Antibody Variable Domains," in Antibody Engineering, Kontermann and Dübel, eds., Chapter 31, pp. 422-439, Springer-Verlag, Berlin (2001). According to one preferred alternative of the invention, the term "CDR" is a CDR as defined by Kabat et al., J. Biol. Chem. 252, 6609-6616 (1977) and Kabat et al., Sequences of protein of immunological interest. (1991). The term "framework region" or "FR region" as used herein, includes the amino acid residues that are part of the variable region, but are not part of the CDRs (e.g., using the Kabat or MacCallum definition of CDRs).

As used herein, the terms "variable region" and "variable domain" are used interchangeably and are common in the art. The variable region typically refers to a portion of an antibody, generally, a portion of a light or heavy chain, typically about the amino-terminal 110 to 120 amino acids or 110 to 125 amino acids in the mature heavy chain and about 90 to 115 amino acids in the mature light chain, which differ extensively in sequence among antibodies and are used in the binding and specificity of a particular antibody for its particular antigen. The variability in sequence is concentrated in those regions called complementarity determining regions (CDRs) while the more highly conserved regions in the variable domain are called framework regions (FR). Without wishing to be bound by any particular mechanism or theory, it is believed that the CDRs of the light and heavy chains are primarily responsible for the interaction and specificity of the antibody with antigen. Preferably, the variable region is a human, humanized, chimeric or primatized variable region. Preferably, the variable region comprises rodent, rabbit or murine CDRs and human framework regions (FRs). It is further possible that the variable region is a primate (*e.g*., non-human primate) variable region. The terms "VL" and "VL domain" are used interchangeably to refer to the light chain variable region of an antibody. The terms "VH" and "VH domain" are used interchangeably to refer to the heavy chain variable region of an antibody.

As used herein, the terms "constant region" and "constant domain" are interchangeable and are common in the art. The constant region is an antibody portion, e.g., a carboxyl terminal portion of a light and/or heavy chain which is not directly involved in binding of an antibody to antigen but which can exhibit various effector functions, such as interaction with an Fc receptor (*e.g*., Fc gamma receptor).

As used herein, the term "heavy chain" when used in reference to an antibody can refer to any distinct type, e.g., alpha (α), delta (δ), epsilon (ε), gamma (γ), and mu (µ), based on the amino acid sequence of the constant domain, which give rise to IgA, IgD, IgE, IgG, and IgM classes of antibodies, respectively, including subclasses of IgG, *e.g*., IgG₁, IgG₂, IgG₃, and IgG₄.

Preferably, the binding molecule or anti-human CD4 antibody for use of the invention comprises a constant region. Preferably, the binding molecule or anti-human CD4 antibody for use of the invention comprises a human IgG constant region, in particular a human IgG₁ or human IgG₄ constant region.

As used herein, the term "light chain" when used in reference to an antibody can refer to any distinct type, e.g., kappa (κ) or lambda (λ) based on the amino acid sequence of the constant domains. Light chain amino acid sequences are well known in the art. In specific embodiments, the light chain is a human light chain.

"Binding affinity" generally refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (*e.g*., binding molecule, such as an antibody, and antigen). The affinity of a molecule X for its partner Y can generally be represented by the dissociation constant (K_{D}). Affinity can be measured and/or expressed in a number of ways known in the art, including, but not limited to, equilibrium dissociation constant (K_{D}), and equilibrium association constant (K_{A}). The K_{D} is calculated from the quotient of k_{off}/kₒₙ, whereas K_{A} is calculated from the quotient of kₒₙ/k_{off}. kₒₙ refers to the association rate constant of, *e.g*., an antibody to an antigen, and k_{off} refers to the dissociation rate constant of, *e.g*., an antibody to an antigen. The kₒₙ and k_{off} can be determined by techniques known to one of ordinary skill in the art, such as surface plasmon resonsance, e.g. using BIAcore^{®}.

Such values can be determined at 37°C. As used herein, a "lower affinity" refers to a larger K_{D}.

Preferably, the binding molecule or antibody for use of the invention binds to human CD4 with a binding affinity K_{D} of 10⁻⁸ M or less, 10⁻⁹ M or less or 10⁻¹⁰ M or less, as determined by surface plasmon resonance, e.g. using BIAcore^{®}, at 37°C.

Particularly preferred examples of anti-CD4 antibodies that may be used in accordance with the present invention are described below.

Preferably, the binding molecule or anti-human CD4 antibody for use of the invention:
i) is selected from the group consisting of Max16H5, OKT4A, OKTcdr4a, cMT-412, YHB.46, particularly wherein said anti-CD4 antibody is Max16H5; or
ii) is antibody 30F16H5; or
iii) is obtainable from a cell line deposited with the ECACC on May 5, 1988 with accession number ECACC 88050502; or
iv) is obtainable from a cell line MAX.16H5/30F16H5 deposited with the DSMZ on December 2, 2011 with Accession number DSM ACC3148; or
v) is antibody 16H5.chimIgG4; or
vi) is obtainable from a cell line CD4.16H5.chimIgG4 deposited with the DSMZ on December 2, 2011 with accession number DSM ACC3147; or
vii) is an antibody comprising the VH and the VK of antibody 16H5.chimlgG4; or viii) is an antibody comprising a VH and a VK of an antibody obtainable from a cell line CD4.16H5.chimlgG4 deposited with the DSMZ on December 2, 2011 with accession number DSM ACC3147; or
ix) is an antibody comprising any combination of a VH selected from SEQ ID NOs: 1-10 and of a VK selected from SEQ ID NOs: 11-20, particularly wherein said combination is selected from VH1/VK1, VH2NK2, VH4NK2 and VH4NK4, especially wherein said combination is VH2NK2, or x) is a mixture of antibodies selected from the antibodies according to i) to ix) above.

The combination of variable regions VH2/VK2 pursuant to SEQ ID NOs: 8 and 18 is particularly preferred for an anti-human CD4 antibody for use herein. SEQ ID No: 8 and 18 represent the VH and VL region, respectively, of the preferred antibody CD4.16H5.chimIgG4 for use in the invention. This antibody is used in Example 1.

Accordingly, preferred anti-CD4 antibodies for use in accordance with the present invention are selected from the group consisting of Max16H5, OKT4A, OKTcdr4a, cMT-412, YHB.46. A particularly preferred anti-CD4 antibody is Max16H5. Cells for the production of Max16H5 have been deposited with the ECACC (European Collection of Cell Cultures) on May 5, 1988 with accession number ECACC 88050502. Said antibody is also disclosed in DE 3919294, which is incorporated by reference herein. As used herein, the antibody "Max16H5" may also be referred to as "Max.16H5", "MAX16H5" or "MAX.16H5", or also "30F16H5" (wherein the latter name is also the name of deposited cells producing said antibody). Max.16H5 may also be obtained from the cell line MAX.16H5/30F16H5 (cf. deposit DSM ACC3148).

A particularly preferred anti-human CD4 antibody for use in the invention is 16H5.chimlgG4. As used herein, said antibody may also be referred to as "16H5.chim" or as "CD4.16H5.chimlgG4" (wherein the latter name is also the name of deposited cells producing said antibody). 16H5.chimlgG4 may be obtained from the cell line CD4.16H5.chimlgG4 (cf. deposit DSM ACC3147).

16H5.chimlgG4 is a chimeric monoclonal antibody comprising a human IgG₄ constant region. 16H5.chimlgG4 is currently in development as Palixizumab^{®}. The use of this antibody is particularly preferred. In some scientific publications, the term "MAX.16H5 IgG4" is used as a synonym for "16H5.chimlgG4".

In detail, certain preferred anti-human CD4 for use in the present invention are e.g. obtainable from any of the following deposits of biological material: i) deposit with the European Collection of Cell Cultures having the accession number ECACC 88050502 on May 5, 1988 (which is e.g. also described in application DE 3919294); ii) deposit "MAX.16H5/30F16H5", accession number DSM ACC3148, deposited with the DSMZ on December 2, 2011; iii) deposit "CD4.16H5.chimIgG4", accession number DSM ACC3147, deposited with the DSMZ on December 2, 2011. All of these deposits involve cells or cell lines, respectively, from which particular anti-CD4 antibodies for use in the context with the present invention may be obtained.

Particularly preferred anti-CD4 antibodies that may be used correspond to the anti-CD4 antibodies described in patent application WO 2012/072268 (cf. particularly the "additional aspect" described therein), which document is incorporated by reference in its entirety herein. This particularly also applies to the embodiments according to items 1-33 disclosed on pages 42 et seqq. of WO 2012/072268 and antibody CD4.16H5.chimIgG4 disclosed therein.

In particular, promising preclinical data are available for antibodies CD4.16H5.chimIgG4 and MAX.16H5 IgG1 in humanized mouse models for the suppression of an allogeneically induced inflammation (GvHD). In particular, proof-of-concept studies have demonstrated the efficacy of the chimeric anti-human CD4 antibody CD4.16H5.chimIgG4 as well as for MAX.16H5 IgG1 in preventing GvHD (i.e. an allogeneically induced inflammation) while maintaining the anti-tumor effect (Kohlhaw K. et al., Transplant.Proc. 2001 33:2371-2373; Laub R. et al., J.Immunol.Methods 2000 246:37-50; Laub R. et al., J. immunol. 2002 169: 2947; Schmidt F. et al.., Cytometry A 2015; 87:334-45; Hilger N. et al., Front Immunol. 2018 Oct 22; 9:2408 and Stahl L. et al. (Front Immunol. 2019 May 24;10:1035). Moreover, these antibodies exhibit an advantageous profile with respect to antigenicity and/or side effects due to the removal of T cell epitopes, as described in WO 2012/072268. Further, the human IgG₄ constant region in CD4.16H5.chimIgG4 is advantageous with respect to side effects.

Accordingly, in a particularly preferred embodiment of the present invention, antibody CD4.16H5.chimIgG4 may be used.

Further alternative anti-CD4 antibodies which may be used in the present invention include, for example, humanized antibody MTRX1011A (Scheerens H. et al., Arthritis Res Ther. 2011; 13(5): R177), monoclonal antibody ibalizumab, antibody Keliximab, and primatized antibody Clenoliximab. Clenoliximab is a chimeric antibody from *Macaca irus* and *Homo sapiens.*

Non-limiting further examples of binding molecules specifically binding to human CD4 which may be used according to the invention are human CD4 ligands and peptides, in particular peptide ligands, including naturally occurring peptide ligands and peptide constructs. Further, human CD4 ligands include aptamers specifically binding to human CD4. Suitable human CD4 ligands, such as small molecules, aptamers, and peptides are known in the art. Peptide ligands specifically binding to human CD4 are disclosed in Sangphukieo A. et al. (PloS one vol. 10,11 e0139562. 30 Oct. 2015, doi:10.1371/journal.pone.0139562). For example, cyclopeptides GA763 or GA190 disclosed in Sangphukieo A. et al. may be used. For example, the peptide or human CD4 ligand may be cyclo(CNSNQIC). Further, the small molecule 4,4'-diisothiocyano-2,2'-dihydrostilbenedisulfonic acid can be used as human CD4 ligand.

According to the invention, the binding molecule for use of the invention is in one preferred embodiment administered intranasally or is administration via inhalation. Thereby, the binding molecule is delivered locally and topically to the respiratory tract, in particular to the lungs. Further, in a preferred embodiment, the binding molecule for use of the invention is comprised in a pharmaceutical composition formulated for intranasal administration and/or for administration via inhalation. The pharmaceutical composition formulated for intranasal administration and/or for administration via inhalation can be administered to the human subject intranasally or via inhalation. Thereby, the binding molecule is delivered locally and topically to the respiratory tract, in particular to the lungs.

For example, in one preferred embodiment, the pharmaceutical composition may be delivered as an aerosol.

Alternatively, the human CD4-specific binding molecule may be administered systemically, for example by intravenous administration, such as intravenous injection.

Suitable pharmaceutical compositions are described below in detail. In one preferred embodiment, the pharmaceutical composition may comprise or consist of a binding molecule described herein in saline solution, in particular in phosphate-buffered saline (PBS). Such pharmaceutical compositions are in particular useful for intranasal administration or for administration via inhalation. Alternatively, such pharmaceutical compositions are useful for systemic administration, in particular for intravenous administration.

The concentration of the binding molecule in a pharmaceutical composition which is a solution may differ, depending e.g. on the binding molecule. In case of an antibody, a concentration of about 0,01 to 100 mg/ml, preferably of between 0,1 to 10 mg/ml may preferably be used.

An amount of binding molecule in a single unit dose may differ, depending e.g. on the binding molecule. In case of an antibody, a single unit does may preferably. comprise between about 0,05 to 500 mg or 0,5 to 50 mg of the binding molecule.

A solution of chimeric antibody 16H5.chimIgG4 in PBS is used in the Example herein at a concentration of 1 mg/ml antibody. The pH is about 7,55.

The binding molecule or pharmaceutical composition may be administered only once to the subject. Alternatively, the binding molecule or pharmaceutical composition may be administered to the subject repeatedly, for example 2, 3, 4, 5, 6, 7, 8, 9, 10 or more times.

Moreover, it is possible to administer the binding molecule or pharmaceutical composition continuously to the subject, for example over a period of 10 minutes or more, 30 minutes or more, or 1 hour or more.

In case of mechanically ventilated subject, it is possible to couple the administration of the binding molecule or pharmaceutical composition to a mechanical ventilation device. Thereby, mechanical ventilation may be maintained during administration of the binding molecule or pharmaceutical composition.

Accordingly, in a more preferred embodiment, the pharmaceutical composition comprises or consists of the binding molecule in saline solution, in particular in phosphate-buffered saline (PBS), and/or
the pharmaceutical composition comprises the binding molecule in a concentration of about 0,1 to 10 mg/ml, and/or
a single unit dose comprises between about 0,5 to 50 mg binding molecule, and/or the binding molecule is antibody 16H5.chimIgG4 or an antibody comprising the VH and the VK of antibody 16H5.chimIgG4, and/or
the binding molecule and/or the pharmaceutical composition is administered once or repeatedly, and/or
the binding molecule and/or the pharmaceutical composition is administered by coupling to a mechanical ventilation device.

In another preferred embodiment, the human subject is mechanically ventilated.

According to the present invention, a mechanically ventilated subject, or ventilated subject, is a subject which is exposed to mechanical ventilation.

Mechanical ventilation aims at maintaining acceptable gas exchange to meet the body's metabolic demands and to minimize adverse effects in its application. The parameters PEEP (positive end-expiratory pressure, to keep alveoli open), mean airway pressure (to promote recruitment (opening) of easily collapsible alveoli and predictor of hemodynamic effects) and plateau pressure (best predictor of alveolar overdistention) are preferably determined.

Any type of ventilator mode may be used. Mechanical ventilation may be invasive or non-invasive.

For example, airway pressure release ventilation (APRV) may be used, in particular when treating ARDS.

Advantages to APRV ventilation include decreased airway pressures, decreased minute ventilation, decreased dead-space ventilation, promotion of spontaneous breathing, almost 24-hour-a-day alveolar recruitment, decreased use of sedation, near elimination of neuromuscular blockade, optimized arterial blood gas results, mechanical restoration of FRC (functional residual capacity), a positive effect on cardiac output increased organ and tissue perfusion and potential for increased urine output secondary to increased kidney perfusion.

Additional measures may be taken in addition to mechanical ventilation. For example, repositioning into the prone position (i.e. face down) can improve oxygenation by relieving atelectasis and improving perfusion. Mechanical ventilation is therefore preferably performed in prone position of the subject. Further, extracorporeal membrane oxygenation (ECMO) may be performed.

It is possible that the subject is ventilated prior, during and/or after administration of the binding molecule for use of the invention. Typically, mechanical ventilation is applied for a longer period of time, such as for at least 1 hour.

For example, the subject is mechanically ventilated for 1, 2, 3, 4, 5, 6, 7, 8, 9, 10, 15, 20, 30, 40, 50, 60, 100 days or more, such as for 5 to 60 days or 10 to 30 days.

For example, the subject is mechanically ventilated for 1, 2, 3, 4, 5, 6, 7, 8, 9, 10, 15, 20, 30, 40, 50, 60, 100 days or more prior to administration of the binding molecule.

Mechanical ventilation may be maintained until ARDS symptoms have improved (i.e. are ameliorated and/or reduced) sufficiently, e.g. until the subject has improved from severe ARDS to moderate ARDS or mild ARDS or until the subject does not exhibit ARDS anymore, or until the subject has improved from moderate ARDS to mild ARDS or until the subject does not exhibit ARDS anymore, or until the until the subject has improved from mild ARDS, until the subject does not exhibit ARDS anymore.

In a yet further embodiment, the present invention relates to a pharmaceutical composition comprising a binding molecule specifically binding to human CD4 for intranasal or inhalation administration comprising at least one binding molecule specifically binding to human CD4 in a single unit dose, and optionally at least one pharmaceutically acceptable excipient, carrier or diluent, and/or an additive for nasal or pulmonary delivery.

In a yet further embodiment, the present invention relates to a pulmonary drug delivery kit comprising: a) an inhaler; and b) the pharmaceutical composition of the present invention.

In a yet further embodiment, the present invention relates to a pharmaceutical package comprising: a) the pharmaceutical composition of the invention; and b) a nebulizer.

For the pharmaceutical composition, the pulmonary drug delivery kit and the pharmaceutical package, the same embodiments as described above for the binding molecules for use of the invention apply.

Moreover, for the binding molecules for use of the invention, the same embodiments as described below for the pharmaceutical composition, the pulmonary drug delivery kit and the pharmaceutical package applies, in particular with respect to administration via inhalation and for intranasal administration.

A binding molecule, such as anti-human CD4 antibody described herein, can be formulated, in one preferred embodiment, for administration via inhalation. For example, the binding molecule can be formulated as an aerosol for topical application, by inhalation or by intranasal administration.

Suitable formulations as an aerosol are known in the art and are described e.g. in U.S. Patent Nos. 4,044,126, 4,414,209 and 4,364,923, which describe aerosols for delivery of a steroid useful for treatment of inflammatory diseases, particularly asthma. These pharmaceutical compositions for administration to the respiratory tract can be in the form of an aerosol or solution for a nebulizer, or as a microfine powder for insufflations, alone or in combination with an inert carrier such as lactose. In such a case, the particles of the formulation will have diameters of less than 50 microns or less than 10 microns. US 5,474,759 discloses aerosol formulations that are substantially free of chlorofluorocarbons. The formulations contain a propellant (such as 1,1,1,2,3,3,3,-heptafluoropropane), a medium-chain fatty acid propylene glycol diester, a medium-chain triglyceride, optionally a surfactant, and optionally auxiliary agents such as antioxidants, preservatives, buffers, sweeteners and taste masking agents.

Accordingly, it is preferred for the pharmaceutical composition, the pulmonary drug delivery kit and the pharmaceutical package, that the pharmaceutical composition is formulated for administration as an aerosol.

It is further preferred for the pharmaceutical composition, the pulmonary drug delivery kit, the pharmaceutical package and uses herein, that the pharmaceutical composition comprises or consists of the binding molecule in saline solution, in particular in phosphate-buffered saline (PBS).

The amount of binding molecule which will be effective in the treatment and/or prevention of a condition will depend on the nature of the disease, and can be determined by standard clinical techniques.

The precise dose to be employed in a composition will also depend on the route of administration, and the seriousness of the infection or disease caused by it, and should be decided according to the judgment of the practitioner and each subject's circumstances. For example, effective doses may also vary depending upon means of administration, target site, physiological state of the subject (including age, body weight and health), whether the subject is human or an animal, other medications administered, or whether treatment is preventive or therapeutic. Usually, the patient is a human but non-human mammals can also be treated. Treatment dosages are optimally titrated to optimize safety and efficacy.

For example, the binding molecule, such as anti-human CD4 antibody described herein, can be formulated in a single unit dose of 1 mg/kg bw or less, of 0.5 mg/kg bw or less, of 0.1 mg/kg bw or less or of 0.05 mg/kg bw or less.

It is further preferred for the pharmaceutical composition, the pulmonary drug delivery kit, the pharmaceutical package and the uses herein, that the pharmaceutical composition comprises the binding molecule in a concentration of about between 0,01 to 100 mg/ml, preferably of between about 0,1 to 10 mg/ml.

For example, it is possible that 1 single dose unit of binding molecule or pharmaceutical composition is administered intranasally or via inhalation to the subject. Alternatively, the binding molecule, such as anti-human CD4 antibody described herein, or pharmaceutical composition, is administered intranasally or via inhalation in multiple doses. Preferably, in case multiple doses are administered, these are administered at least 2 hours apart from each other. For example, 2, 3, 4, 5, 6, 7, 8, 9, 10 or more single unit doses may be administered. For example, the single unit doses may be administered daily, twice daily, every 2, 3 or 4 days, weekly or monthly.

Accordingly, it is further preferred for the pharmaceutical composition, the pulmonary drug delivery kit, the pharmaceutical package and the uses herein, that the single unit dose comprises between about 0,05 to 500 mg or between about 0,5 to 50 mg binding molecule, more preferably between about 0,5 to 50 mg binding molecule, in particular wherein the binding molecule is an anti-human CD4 antibody.

Moreover, it is preferred for the pharmaceutical composition, the pulmonary drug delivery kit, the pharmaceutical package and the uses herein, that the binding molecule is antibody 16H5.chimIgG4 or an antibody comprising the VH and the VK of antibody 16H5.chimIgG4.

It is further preferred for the pharmaceutical composition, the pulmonary drug delivery kit, the pharmaceutical package and the uses herein, that the pharmaceutical composition is in a container which allows coupling to a mechanical ventilation device, or wherein the nebulizer or inhaler allows coupling to a mechanical ventilation device.

The binding molecule is preferably in a pharmaceutical composition for intranasal or inhalation administration. Suitable formulations for pulmonary drug delivery and/or devices for delivering binding molecule, such as an antibody, can be used and are known in the art. For example, aerosol pharmaceuticals compositions are provided herewith for the delivery of a binding molecule, such as an antibody, optionally combined with an additional active agent to the respiratory tract.

Pulmonary drug delivery may be achieved by administration via inhalation, and administration by inhalation herein may be oral and/or nasal.

According to the present invention, administration is in one preferred embodiment via inhalation or by intranasal administration.

Examples of pharmaceutical devices for administration via inhalation or intranasal administration, for pulmonary delivery, as well as corresponding methods and uses, include metered dose inhalers (MDIs), dry powder inhalers (DPIs), and nebulizers. Exemplary delivery systems by inhalation which can be adapted for delivery of a binding molecule, in particular antibody, and optionally at least one further active agent, to the subject are described in, for example, US 5,756,353; US 5,858,784; WO98/31346; WO98/10796; WO00/27359; WO01/54664; and WO02/060412. Other aerosol pharmaceutical compositions and devices that may be used for delivering the binding molecule via inhalation or intranasal administration are described in US 6,294,153; US 6,344,194; US 6,071,497, WO02/066078; WO02/053190; WO01/60420; and WO00/66206.

Pressurized metered dose inhalers (pMDIs) are the most commonly used inhaler worldwide (see e.g. WO2006/122257 for details). The aerosol is created when a valve is opened (usually by pressing down on the propellant canister), allowing liquid propellant to spray out of a canister. Typically, a drug or therapeutic is contained in small particles (usually a few microns in diameter) suspended in the liquid propellant, but in some formulations, the drug or therapeutic may be dissolved in the propellant. The propellant evaporates rapidly as the aerosol leaves the device, resulting in small drug or therapeutic particles that are inhaled. Propellants typically used in such pMDIs include but are not limited to hydrofluoroalkanes (HFAs). A surfactant may also be used, for example, to formulate the drug or therapeutic, with pMDIs. Other solvents or excipients may also be employed with pMDIs, such as ethanol, ascorbic acid, sodium metabisulfate, glycerin, chlorobutanol, and cetylpyridium chloride. Such pMDIs may further include add-on devices such as, for example, spacers, holding chambers and other modifications.

Nebulizers produce a mist of drug-containing liquid droplets for inhalation(see e.g. for details WO2006/122257). They are usually classified into two types: ultrasonic nebulizers and jet nebulizers. A type of nebulizer is also available which does not require ultrasound or air pressure to function. Single breath atomizers have also been developed (e.g., Respimat^{®}), which is used to deliver a drug in a single inhalation and may be preferred because of less contamination. Jet nebulizers use a source of pressurized air to blast a stream of air through a drug-containing water reservoir, producing droplets in a complex process involving a viscosity-induced surface instability that leads to nonlinear phenomena in which surface tension and droplet breakup on baffles play a role. Ultrasonic nebulizers produce droplets by mechanical vibration of a plate or mesh. In either type of nebulizer, the drug is usually contained in solution in the liquid in the nebulizer and so the droplets being produced contain drug in solution. However, for some formulations (e.g., Pulmicort) the drug is contained in small particles suspended in the water, which are then contained as particles suspended inside the droplets being produced. Certain excipients are usually included in formulations suitable for nebulization, such as sodium chloride (e.g., to maintain isotonicity), mineral acids and bases (e.g., to maintain or adjust pH), nitrogen headspace sparging, benzalkonium chloride, calcium chloride, sodium citrate, disodium edtate, and polysorbate 80.

The third type of inhaler is the dry powder inhaler (DPI) (see e.g. WO2006/122257 for details). In DPIs, the aerosol is usually a powder, contained within the device until it is inhaled. The therapeutic or drug is manufactured in powder form as small powder particles (usually a few millionths of a meter, or micrometers, in diameter). In many DPIs, the drug or therapeutic is mixed with much larger sugar particles (e.g., lactose monohydrate), that are typically 50-100 micrometers in diameter. The increased aerodynamic forces on the lactose/drug agglomerates improve entrainment of the drug particles upon inhalation, in addition to allowing easier filling of small individual powder doses. Upon inhalation, the powder is broken up into its constituent particles with the aid of turbulence and/or mechanical devices such as screens or spinning surfaces on which particle agglomerates impact, releasing the small, individual drug powder particles into the air to be inhaled into the lung. The sugar particles are usually intended to be left behind in the device and/or in the mouth-throat.

An "aerosol pharmaceutical composition" or "aerosol composition" is understood as a binding molecule described herein in a form or formulation that is suitable for administration via inhalation and/or intranasal administration as an aerosol. The aerosol composition may be in the dry powder form, it may be a solution, suspension or slurry to be nebulized, or it may be in admixture with a suitable low boiling point, highly volatile propellant. It is to be understood that more than one binding molecule and optionally other active agents or ingredients may be incorporated into the aerosolized pharmaceutical composition or aerosol pharmaceutical composition.

In one preferred embodiment, an aerosol pharmaceutical composition is provided. The aerosol pharmaceutical composition can be a composition comprising aerosolized binding molecule for use herein or a composition comprising a binding molecule for use herein in a formulation suitable for aerosolization. The binding molecule for use herein, in particular antibody, may be formulated in combination with an additional active agent, and the combination formulation is suitable for aerosolization. Alternatively, the binding molecule for use herein and optionally an additional active agent may be formulated separately, such that they will be combined after aerosolization occurs or after being administered to a subject.

In another preferred embodiment, a nebulization pharmaceutical composition is provided. The nebulization pharmaceutical can be a composition comprising a nebulized a binding molecule for use herein or a composition comprising a binding molecule for use herein in a formulation suitable for nebulization. Similarly, the binding molecule for use herein may be formulated in combination with an additional active agent, and the combination formulation is suitable for nebulization. Alternatively, the binding molecule for use herein and optionally an additional active agent may be formulated separately, such that they will be combined after nebulization occurs or after being administered to a subject.

A nebulizer for use herein is preferably selected from a jet air nebulizer, an ultrasonic nebulizer, a vibrating mesh nebulizer and a Shockwave nebulizer. Nebulized solutions may be prepared by aerosolizing commercially available active agent formulation solutions. These solutions may be delivered by a jet nebulizer. Other methods for delivery of solutions, suspensions of slurries are described in US 5,672,581. A device that uses a vibrating, piezoelectric member is described US. 5,586,550.

The pharmaceutical package may further comprise an additional active agent in addition to the binding molecule. The pharmaceutical package may also comprise instructions for use.

A binding molecule for use of the present application may be produced or processed in bulk and packaged in an ampule or container made of a suitable material (e.g., glass or plastic) at different doses. The antibody may be stable in a formulation at a concentration ranging from 0,01 to 100 mg/ml.

An additional active agent (or an active agent in addition to the binding molecule for use herein) herein can be another antibody therapeutic, preferably an anti-human IL-6 receptor antibody, in particular tocilizumab, a sympathomimetic (e.g., albuterol), an antibiotic (e.g., tobramycin), a deoxyribonuclease (e.g., pulmozyme), an anticholinergic drug (e.g., ipratropium bromide), a corticosteroid (e.g., dexamethasone), a β-adrenoreceptor agonist, a leukotriene inhibitor (e.g., zileuton), a 5 Lipoxygenase inhibitor, a PDE inhibitor, a CD23 antagonist, an IL-13 antagonist, a cytokine release inhibitor, or an anti-inflammatory agent, such as a corticosteroid or an NSAID. As used herein, an active agent may also be referred to as a therapeutic or a drug.

A preferred formulation or pharmaceutical composition suitable for aerosolization or nebulization of binding molecule herein is in physiologic osmolarity (e.g., between 280 and 320 mM) and/or at a suitable pH (e.g., pH 6 to 8).

Preferably, the pH is between about 6,8 and 8, such as between about 7,2 to 7,8.

Other pharmaceutically acceptable carriers may also be used in a pharmaceutical composition of the present application. The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent or encapsulating material, involved in carrying or transporting the binding molecule from one organ, or portion of the body, to another organ, or portion of the body. Each carrier must be "acceptable" in the sense of being compatible with the other ingredients of the formulation and not injurious to the subject. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Methods of preparing these formulations or compositions include the step of bringing into association a binding molecule for use of the present application with the carrier and, optionally, excipients, diluents, and/or additives. In general, the pharmaceutical compositions are prepared by uniformly and intimately bringing into association a binding molecule herein with liquid carriers, or finely divided solid carriers, or both, and then, if necessary, shaping the product.

The binding molecules for use herein and the pharmaceutical compositions herein, such as aerosol or nebulization pharmaceutical compositions, can be combined with one or more other therapies, concurrently or sequentially. The one or more other therapies may be administered via inhalation or intranasal administration or via other routs of administration, such as via systemic, intravenous or oral routes, e.g. in case of immunosuppressants, such as corticosteroids.

In a yet further embodiment, the present invention relates to a method for preventing, ameliorating and/or treating at least symptom of a disease associated with inflammation of the respiratory tract and/or immunologically induced injury of the respiratory tract in a subject need thereof, comprising administering to the subject an effective amount of a binding molecule specifically binding to human CD4, wherein the binding molecule is administered intranasally and/or via inhalation and/or systemically.

For the methods herein, the same embodiments as described above for the binding molecules for use, the pharmaceutical composition, the pulmonary drug delivery kit and the pharmaceutical package described herein apply.

As used herein, the term "treat," "treating," and "treatment" refer to therapeutic or preventative measures described herein. The methods and uses of "treatment" employ administration of a binding molecule to a subject having a disease or disorder, or predisposed to having such a disease or disorder, in order to prevent, cure, delay, reduce the severity of, or ameliorate one or more symptoms of the disease or disorder or recurring disease or disorder, or in order to prolong the survival of a subject beyond that expected in the absence of such treatment.

In particular, "ameliorate", "ameliorating" and "amelioration" employ administration of a binding molecule to a subject having a disease or disorder, or predisposed to having such a disease or disorder, in order to reduce the severity and/or duration of one or more symptoms of the disease or disorder or recurring disease or disorder, or in order to prolong the survival of a subject beyond that expected in the absence of such treatment.

As used herein, the term "effective amount" in the context of the administration of a therapy to a subject refers to the amount of a therapy that achieves a desired preventive, ameliorating or therapeutic effect.

As used herein, the term "subject" includes any human or non-human mammalian animal. Preferably, the subject is a human.

In general, the disclosure is not limited to the particular methodology, protocols, and reagents described herein because they may vary. Further, the terminology used herein is for the purpose of describing particular embodiments only and is not intended to limit the scope of the present disclosure. As used herein and in the appended claims, the singular forms "a", "an", and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the disclosure.

The term "about" in the context of a value refers to the value ± 10%, preferably the value ± 5%.

### EXAMPLES

### Example 1: Assessment of the in vivo effect of the pulmonary delivery of antibody 16H5.chimIgG4 in humanized mice infected with SARS-CoV-2

The SARS-CoV-2-specific human innate, humoral, and cellular immune responses are assessed in humanized mice (mice with a human immune system [HIS mice]) with functional human CD4⁺ -T and B-cells. These mice are for example described in Sharma A. et al (2016, Journal of Virology, 90: 5068-5074). In particular, these mice were generated in Sharma et al. by introduction of HLA class II genes, various human cytokines, and human B cell activation factor into immunodeficient NOD scid gamma (NSG) mice by the use of an adeno-associated virus vector, followed by engraftment of human hematopoietic stem cells. These HIS mice possess functional human CD4⁺ -T and B-cells.

In particular, mice can be generated as described in Sharma A. et al (supra). In detail, NSG (NOD.Cg-*Prkdc^{scid} IL2rg^{tmWjl}*/*Sz*) mice can be purchased from a suitable source (such as The Jackson Laboratory) and maintained under specific-pathogen-free conditions.
HIS mice that possess functional human CD4⁺ T and B cells through AAV9-mediated modification with human cytokine genes are used in this study (see reference (16) in Sharma et al.). To obtain HIS mice, young NSG mice (2 to 3 weeks old) can be first inoculated with 1 x 10¹¹ genomic copies (GC) of AAV9-HLA class II (DR1 or DR4) (5 x 10¹⁰ GC intrathoracically and 5 x 10¹⁰ GC intraperitoneally), together with intraperitoneal injection of 5 x 10¹⁰ GC of AAV9-B cell-activating factor belonging to the tumor necrosis factor family (BAFF) and 5 x 10⁹ GC of AAV9-human cytokines (AAV9-interleukin- 3 [IL-3], AAV9-IL-4, AAV9-IL-7, AAV9-IL-15, AAV9-granulocyte macrophage colony-stimulating factor, and AAV9-macrophage colony-stimulating factor). Two weeks later, the mice are subjected to sublethal irradiation, followed by intravenous administration of 1 x 10⁵ human CD34⁺ hematopoietic stem cells (HSCs) derived from human fetal liver.

Since these HIS mice receive only HLA class II and not HLA class I, the human CD8 cells in these mice are non-functional. NSG-mice not infected with the AAV9 vector or transfused with human HSCs can be used as negative controls.

Rapid host responses to intranasal challenge with SARS-CoV-2 can be assessed as follows:
To evaluate the host responses against SARS-CoV-2 pulmonary infection, HIS and NSG mice are challenged with SARS-CoV-2 (for example, 10⁶ PFU in 50 µl phosphate-buffered saline (PBS) per mouse) by intranasal inoculation. Three days later the mice are sacrificed, and lung homogenates are stored at -80°C or in TRIzol reagent (Invitrogen) for RNA isolation. The SARS-CoV-2 titers in lung homogenates can be quantified by plaque assay and expressed as the number of PFU per lung as previously described in Sharma et al.

RNA is converted to cDNA using random hexamers and reverse transcription (RT) reagents (Applied Biosystems). Quantification of the relative levels of expression of mRNA for murine and human mRNAs of interest can be performed by qRT-PCR. Lung histopathology and differential cell counts in bronchioalveolar lavage (BAL) fluid can be evaluated as described in Sharma et al.

Anti-SARS-CoV-2 adaptive immune responses can be assessed as follows:
HIS and NSG mice can be infected intranasally with SARS-CoV-2 (for example, 10⁶ PFU per mouse) and can be infected again e.g. 1 and 6 weeks later. Serum is collected e.g. at 2, 4, and 7 weeks following initial SARS-CoV-2 administration. BAL fluid is collected by intratracheal instillation and aspiration of 0.5 ml PBS. Anti-SARS-CoV-2 human IgG in serum and human IgA in BAL fluid can be assessed by enzyme-linked immunosorbent assay (ELISA; Bio-Rad Laboratories) as described in Sharma et al.. SARS-CoV-2-neutralizing antibody titers in serum can be quantified by plaque assay, as described in Sharma et al. The SARS-CoV-2-specific cellular immune response can determined by an enzyme-linked immunosorbent spot (ELISPOT) assay for human gamma interferon (IFN-y) secreted from splenocytes following stimulation with heat-inactivated SARS-CoV-2. Mouse splenocytes are isolated e.g. at 7 weeks as a single-cell suspension and cultured in RPMI medium supplemented with 2% fetal bovine serum, 10 mM HEPES (pH 7.5), and 10 µM β-mercaptoethanol in 96-well MultiScreen-HA plates coated with of 10 µg/ml of anti-human IFN-γ capture antibody. The splenocytes (10⁶/well) can be treated with 10 µg/ml of heat-inactivated purified SARS-CoV-2 for 24 h. The wells are washed, incubated with a biotinylated anti-human IFN-γ antibody (1 µg/ml) for 2 h followed by avidin-horseradish peroxidase (1:1,000), and developed using angiotensin-converting enzyme (ACE) substrate. The number of distinct spots can be counted under a stereomicroscope.

The *in vivo* effect of the pulmonary delivery of anti-human CD4-specific antibody 16H5.chimIgG4 (disclosed in WO 2012/072268) is studied in the humanized mice infected with SARS-CoV-2. In short, a pharmaceutical composition for use of the present invention is prepared: a sterile solution of antibody 16H5.chimIgG4 in PBS (concentration: 1 mg/ml), pH 7,55 ± 0,2. The pharmaceutical composition is administered to the humanized mice infected with SARS-CoV-2 by intranasal administration at one or more time points after SARS-CoV-2 infection.

The analysis of the SARS-CoV-2-infected HIS mice is expected to show that the pulmonary, intranasal administration of the anti-human CD4-specific antibody 16H5.chimIgG4 results in an amelioration of local inflammatory reactions in the lungs as compared to control animals, in particular by showing an anti-inflammatory effect on T helper cells and/or macrophages and/or an amelioration of signs of a lymphocytic endotheliitis of the lung and/or an amelioration of symptoms of acute respiratory distress.

## Claims

1. A binding molecule specifically binding to human CD4 for use in the prevention, amelioration and/or treatment of at least symptom of a disease associated with inflammation of the respiratory tract and/or immunologically induced injury of the respiratory tract in a human subject, wherein the binding molecule is administered intranasally and/or via inhalation and/or systemically.

2. The binding molecule for use of claim 1, wherein the disease associated with inflammation of the respiratory tract and/or immunologically induced injury of the respiratory tract is selected from Acute Respiratory Distress Syndrome (ARDS), asthma, chronic obstructive pulmonary disease (COPD), lung fibrosis, pulmonary Graft-versus-Host-Disease (GvHD), aspiration (inhalation) of food components into the lungs, burns, pregnancy complications, in particular selected from amniotic fluid embolism, pre-eclampsia, infection of uterine tissue before, during or after a miscarriage (septic abortion), breast injury, in particular lung contusion, coronary artery bypass surgery, drowning, inflammation of the pancreas (pancreatitis), inhalation of large quantities of smoke, inhalation of other toxic gases, damage to the lungs through inhalation of highly concentrated oxygen, life threatening or serious injuries, overdose of substances in particular selected from heroin, methadone, propoxyphene or acetylsalicylic acid, pneumonia, prolonged or threateningly low blood pressure (circulatory shock), pulmonary embolism, severe, systemic infection (sepsis), stroke or cramping seizure, transfusions of more than about 15 blood preserves in a short time, infection or sepsis with extra-pulmonary focus, disruption of microcirculation (shock), systemic inflammatory response syndrome (SIRS), extrapulmonary organ failure, and metabolic derailment.

3. The binding molecule for use of claim 2, wherein the ARDS is caused by a pathogen, in particular SARS-CoV2, pneumonia, aspiration, inhalational lung injury, lung contusion, chest trauma, near-drowning, sepsis, shock, pancreatitis, trauma, fat embolism, cardiopulmonary bypass, transfusion-related acute lung injury (TRALI), burns, or increased intracranial pressure.

4. The binding molecule for use of any one of claims 1-3, wherein
i) the binding molecule specifically binding to human CD4 is selected from an anti-human CD4 antibody, a human CD4 ligand or a peptide, and/or
ii) the binding molecule specifically binding to human CD4 is a monoclonal antibody or an antigen-binding fragment thereof, or is a human, humanized, primatized or a chimeric antibody, and/or
iii) the binding molecule specifically binding to human CD4 is a non-depleting anti-human CD4 antibody.

5. The binding molecule for use of any one of claims 1-4, wherein the binding molecule specifically binding to human CD4 is selected from an anti-human CD4 antibody, in particular wherein the anti-human CD4 antibody:
i) is selected from the group consisting of Max16H5, OKT4A, OKTcdr4a, cMT-412, YHB.46, particularly wherein said anti CD4 antibody is Max16H5;
or
ii) is antibody 30F16H5;
or
iii) is obtainable from a cell line deposited with the ECACC on May 5, 1988 with accession number ECACC 88050502;
or
iv) is obtainable from a cell line MAX.16H5/30F16H5 deposited with the DSMZ on December 2, 2011 with Accession number DSM ACC3148;
or
v) is antibody 16H5.chimIgG4;
or
vi) is obtainable from a cell line CD4.16H5.chimIgG4 deposited with the DSMZ on December 2, 2011 with accession number DSM ACC3147;
or
vii) is an antibody comprising the VH and the VK of antibody 16H5.chimIgG4;
or
viii) is an antibody comprising a VH and a VK of an antibody obtainable from a cell line CD4.16H5.chimIgG4 deposited with the DSMZ on December 2, 2011 with accession number DSM ACC3147;
or
ix) is an antibody comprising any combination of a VH selected from SEQ ID NOs: 1-10 and of a VK selected from SEQ ID NOs: 11-20, particularly wherein said combination is selected from VH1 pursuant to SEQ ID NO: 10/VK1 pursuant to SEQ ID NO: 20, VH2 pursuant to SEQ ID NO: 8/VK2 pursuant to SEQ ID NO: 18, VH4 pursuant to SEQ ID NO: 4/VK2 pursuant to SEQ ID NO: 18 and VH4 pursuant to SEQ ID NO: 4/VK4 pursuant to SEQ ID NO: 14, especially wherein said combination is VH2 pursuant to SEQ ID NO: 8 and VK2 pursuant to SEQ ID NO: 18.

6. The binding molecule for use of any one of claims 1-5, wherein the binding molecule is comprised in a pharmaceutical composition formulated for intranasal administration and/or for administration via inhalation,
preferably
wherein the binding molecule and/or the pharmaceutical composition is administered as an aerosol, and/or
wherein pharmaceutical composition comprises or consists of the binding molecule in saline solution, in particular in phosphate-buffered saline (PBS), and/or
wherein pharmaceutical composition comprises the binding molecule in a concentration of about 0,1 to 10 mg/ml, and/or
wherein a single unit dose comprises between about 0,5 to 50 mg binding molecule, and/or
wherein the binding molecule is antibody 16H5.chimIgG4 or an antibody comprising the VH and the VK of antibody 16H5.chimlgG4, and/or
wherein the binding molecule and/or the pharmaceutical composition is administered once or repeatedly, and/or
wherein the binding molecule and/or the pharmaceutical composition is administered by coupling to a mechanical ventilation device.

7. The binding molecule for use of claim 3, wherein the ARDS is caused by SARS-CoV2 and/or wherein the human subject is diagnosed to have a SARS-CoV2 infection and/or wherein the human subject suffers from SARS-CoV2.

8. The binding molecule for use of any one of the preceding claims, wherein
a) the binding molecule specifically binding to human CD4 is for use in the amelioration and/or treatment of at least symptom of Acute Respiratory Distress Syndrome (ARDS), and/or
b) the human subject suffers from ARDS, and/or
c) the human subject exhibits preexisting endotheliitis and/or preexisting endothelial dysfunction, in particular of the respiratory tract, and/or
d) the human subject exhibits one or more risk factors selected from male sex, age of 60 years or more, smoking, elevated blood pressure, diabetes, obesity and a cardiovascular disease.

9. The binding molecule for use of any one of the preceding claims, wherein the human subject is mechanically ventilated.

10. The binding molecule for use of any one of the preceding claims, wherein
a) the administration of the binding molecule decreases mortality, and/or
b) the administration of the binding molecule reduces the duration of mechanical ventilation of the subject, and/or
c) the administration of the binding molecule specifically modulates immune responses to inflammatory cells in the respiratory tract, in particular wherein the inflammatory cells are T helper cells and/or macrophages, and/or
d) the administration of the binding molecule ameliorates and/or suppresses local immune reactions in the respiratory tract, and/or
e) the administration of the binding molecule ameliorates and/or suppresses endotheliitis, in particular lymphocytic endotheliitis, in the respiratory tract.

11. The binding molecule for use of any one of the preceding claims, wherein the at least symptom of the disease associated with inflammation of the respiratory tract and/or immunologically induced injury of the respiratory tract is selected from acute severe respiratory distress, hypoxemia and bilateral diffuse pulmonary infiltrates.

12. The binding molecule for use of claim 11, wherein the treatment ameliorates or treats at least symptom selected from acute severe respiratory distress, hypoxemia and bilateral diffuse lung infiltrates.

13. A pharmaceutical composition comprising a binding molecule specifically binding to human CD4 for intranasal or inhalation administration comprising at least one binding molecule specifically binding to human CD4 in a single unit dose, and optionally at least one pharmaceutically acceptable excipient, carrier or diluent, and/or an additive for nasal or pulmonary delivery.

14. A pulmonary drug delivery kit comprising: a) an inhaler; and b) the pharmaceutical composition of claim 13.

15. A pharmaceutical package comprising: a) the pharmaceutical composition of claim 13; and b) a nebulizer.

16. The pharmaceutical composition of claim 13, the pulmonary drug delivery kit of claim 14, or the pharmaceutical package of claim 15,
wherein the pharmaceutical composition is formulated for administration as an aerosol, and/or
wherein the pharmaceutical composition comprises or consists of the binding molecule in saline solution, in particular in phosphate-buffered saline, and/or wherein the pharmaceutical composition comprises the binding molecule in a concentration of about 0,1 to 10 mg/ml, and/or
wherein the single unit dose comprises between about 0,5 to 50 mg binding molecule, and/or
wherein the binding molecule is antibody 16H5.chimIgG4 or an antibody comprising the VH and the VK of antibody 16H5.chimIgG4, and/or
wherein the pharmaceutical composition is in a container which allows coupling to a mechanical ventilation device, or wherein the nebulizer or inhaler allows coupling to a mechanical ventilation device.
